# EUROPEAN PATENT APPLICATION

(11) **EP 2 481 298 A2**
(43) Date of publication of application: **01.08.2012**
(21) Application number: 10818442.5
(22) Date of filing: 23.09.2010
(51) Int. Cl.: A23L 1/30, A23L 2/02, A23L 2/52, A23L 2/84, A61K 36/185, A61K 36/63, A61K 36/31, A61P 9/12, A61P 3/06

(54) **USE OF PLANT EXTRACTS AS PREBIOTICS, COMPOSTIONS AND FOODS CONTAINING SUCH EXTRACTS**

(30) Priority: 23.09.2009 ES 200901931
(71) Applicant: Probelte Pharma, S.A., 30100 Espinardo Murcia (ES)
(72) Inventor: PEÑALVER MELLADO, Marcos, E-30007 Murcia (Murcia) (ES); LÓPEZ MAS, José, A., E-30840 Alhama de Murcia (Murcia) (ES); STREITENBERGER, Sergio, A., E-30120 El Palmar (Murcia) (ES); MARTÍNEZ ORTIZ, Pedro, E-30150 La Alberca (Murcia) (ES)
(74) Representative: Torner Lasalle, Elisabet
(86) International application number: PCT/ES2010/000389
(87) International publication number: WO 2011/036316

(57) **Abstract**

The present invention claims the use of olive (Olea europaea) and pomegranate (Punica granatum) plant extracts and various combinations of said extracts, some additionally including a probiotic, which allow a synergistic effect between the bioactive components thereof, for the purpose of preparing dietary supplements, functional foods/beverages, food additives and drugs for use in caring for human health.

## Description

### Field of the invention

The present invention claims the use of plant extracts and various combinations of these extracts, some additionally including a probiotic, which allow a synergistic effect between the bioactive components thereof, for the preparation of dietary supplements, functional foods/beverages, food additives and drugs to be used in human health care.

More specifically, the invention describes the use of an olive extract standardized to hydroxytyrosol (MEDITEANOX^{®}) and a pomegranate extract standardized to punicalagins (POMANOX ®) as a prebiotic ingredient. Within the scope claimed also describes the use of mentioned olive and pomegranate extracts with prebiotic properties in combination with a probiotic for the production of a symbiotic (defined as the food that contains a specific mixture of probiotic bacteria and prebiotic substances).

Furthermore, the invention discloses the combinations of the pomegranate extract and/or olive extracts with prebiotic effect or other prebiotics or a dietary fibre with a vegetal extract containing glucosinolates, such as broccoli extract, when finding that a selective stimulation of certain populations of colonic microflora, such as Bifidobacterium and Lactobacillus, improves the bioavailability of isothiocyanates in our body; these isothocyanates are formed from their precursors, glucosinolates, in the colon.

It must be noted that when in this invention the use of a food is referred, it is understood to include also dietary supplements, functional foods, food additives or drugs.

### Background of the invention

The digestive tract of an adult contains a flora composed of approximately 1014 microorganisms, with about 400-500 different bacterial species. The dominant population consists of strictly anaerobic bacteria: Bacteroides, Bifidobacterium, Eubacterium and Peptostreptococcus. Subdominant flora includes bacteria belonging to the genera Streptococcus and Lactobacillus, and to a lesser extent, Enterococcus, Clostridium and yeasts. Most of these species has a beneficial role, but others are potentially pathogenic, as some species of *Clostridium,* although the small number and competition with other bacteria prevent their proliferation and their pathogenic action.

The gastrointestinal flora in the body performs different functions. Its importance is primarily defensive, since it prevents the colonization by pathogenic microorganisms and modulates the immune system by inducing the production of tolerance and non-inflammatory immunostimulants. Moreover, the flora metabolism is an important source of energy for the gut wall due to the fermentation of carbohydrates to organic acids, also produces the synthesis of certain vitamins, such as K and some B. No less important is the role played by the microorganisms in the regulation of intestinal transit.

The stability of the gastrointestinal flora depends on several parameters: physiological, such as age, menopause and stress, certain diseases, especially diarrheal, colitis and Crohn's disease and consumption of drugs, especially antibiotics, and nutritional. In this latter aspect comes into play is where the consumption of probiotics, prebiotics and symbiotics. Probiotics are non-pathogenic microorganisms that, when ingested, exert a positive influence on host health or physiology. Commercial preparations of probiotics are primarily formed by bacteria of the genus Bifidobacterium and / or Lactobacillus.

Probiotics are non-pathogenic microorganisms that, when ingested, exert a positive influence on host health or physiology. Commercial preparations of probiotics are primarily formed by bacteria of the *Bifidobacterium* and / or *Lactobacillus* genus.

A prebiotic is defined as a no digestible food ingredient that has the potential to improve host health by selectively stimulating the growth and / or activity of one or a limited number of bacteria in the colon (Gibson et al., 2004). For a prebiotic ingredient is considered it must meet three requirements (Collins and Gibson, 1999): i) that is not absorbed or hydrolyzed in the stomach, ii) is a selective substrate for one or a limited number of potentially beneficial bacteria in the colon, stimulating their growth and / or metabolic activity; iii) that as a result of the above, is capable of altering the composition of intestinal flora to a composition richer in bacteria.

A symbiotic food is defined as one containing a specific mixture of probiotic bacteria and prebiotic substances.

Various substances have been shown to be active as prebiotics, among which inulin and fructooligosaccharides (FOS). These significantly affect the composition of intestinal flora, reduce the incidence of gastrointestinal infections, respiratory and atopic dermatitis, and also produce an increase in the population of Bifidobacterium in the colon. Other oligo and polysaccharides can act as prebiotics.

There is extensive literature that defines the beneficial health effects of consumption of prebiotic ingredients (see review Venter, 2007), developed in vitro assays and in vivo animal models and humans. Research has led to the identification of biomarkers able to demonstrate the effects of prebiotics. These markers are: i) changes in gastrointestinal flora and the overall metabolism of the gastric environment, especially the production of organic acids, ii) modulation of the immune system, assessing inflammatory and immune globulins iii) increase the absorption of minerals in the colon, such as calcium, zinc or magnesium iv) regulation of lipid metabolism, lowering cholesterol, v) prevention of colon cancer. This last point has attracted considerable interest in recent years and several publications demonstrate the preventive effect of probiotics in this cancer (see reviews by Pool-Zoobel, 2005 and Liong, 2008).

Sometimes part of the effect may occur at the level of the intestine itself, but this does not imply that the effect can not be transmitted to other target organs of the human organism. As an example, dietary fibre (e.g. soluble fibre) can act sequestering bile acids in the intestine itself, which leads to not be reabsorbed and thus the liver responds by using the endogenous cholesterol to synthesize more fatty acids in bile send the intestine. But a second mechanism of action means that when the soluble fibre undergoes fermentation in the gut increases the production of short chain fatty acids such as propionate. Propionate is absorbed in the colon through the portal vein and has been shown to inhibit HMGR in the liver.

The application GB 2198126 describes the use T3 carbohydrate D-tagatose as a prebiotic food component. Consumption induces the production of butyrate in the colon, which may have a protective effect against colon cancer.

The application ES 2278021 T3 describes the use of a composition comprising a prebiotic to reduce the inflammatory process and abnormal activation of non-specific immune parameters. The prebiotic comprises an oligosaccharide produced from glucose, galactose, xylose, maltose, sucrose, lactose, starch, xylan, hemicelluloses, inulin, gum (e.g. gum acacia) or a mixture thereof.

The application ES 2320988 T3 describes a method for preventing or alleviating the symptoms of malabsorption through the gastrointestinal tract by administering a probiotic that can be inulin, modified, hydrolyzed inulin, fructo-oligosaccharides, galactomannan and hydrolysates, arabinogalactan and hydrolysates thereof, trans-galacto-oligosaccharides, rhamnose, pectin and hydrolysed pectin, resistant starch and hydrolysates thereof, indigestible dextrin and hydrolysates thereof, indigestible polydextrose, beta-glucan and hydrolysates thereof, and combinations of these oligo and polysaccharides.

The application WO 02/091833 describes a pharmaceutical composition comprising a probiotic, a prebiotic and a microorganism with stability at alkaline pH and high urea activity. The prebiotic oligosaccharide is described, inulin, lactulose and other plant fibres.

The application U.S. 2009/0022849 A1 describes the prebiotic effect of red fruit and red fruit juices, such as currant, cranberry and pomegranate, and its use to promote growth of beneficial bacteria in the gastrointestinal flora, such as Bifidobacterium and Lactobacillus, and inhibit the growth of harmful bacteria in the gastrointestinal flora, such as Clostridium and Bacteroides. However, the authors do not indicate what the compounds in the berries are and their juices that provide properties to promote growth of beneficial bacteria and inhibit the growth of harmful bacteria, and therefore do not proposed the standardization of the juices to compounds able to promote these effects.

It is described that the ellagitannins, among which are the punicalagins, and ellagic acid, which can be liberated by the hydrolysis of punicalagins, are metabolized in part by the human colonic microbiota. As a result of its metabolism by the colonic microbiota are a series of compounds that are chemically dibenzopiranonas of which the most significance in vivo are known as A and B urolithins.

However, is completely unknown what are the microbiota microorganisms responsible for the production of urolithins and it is certainly of interest identification and use as probiotics to boost the production of urolithins given that described the effect of urolithins in the chemoprevention of cancer.

In the context of chemoprevention defined as the use of drugs or other substances to try to reduce the risk of cancer, retard or prevent the return, the patent US5411986 discloses that isothiocyanates such as sulforaphane, which was isolated by the authors from broccoli, a potent inducer of key enzymes of liver detoxification phase II can be used in the field of oncology for its chemo preventive properties.

The US5725895 discloses a method for preparing foods rich in glucosinolates and/or isothiocyanates from cruciferous seeds and sprouts obtained from them.

The cruciferous vegetables, remarkably broccoli are high in glucosinolates (they are chemically thioglucosides) and, in this vegetables, are accompanied by the enzyme myrosinase (which shows thioglucosidase activity). When the plant is cut or chewed the enzyme catalyzes the conversion of different glucosinates into corresponding aglycones that are known as isothiocyanates and are bioactive products. For example, the glucorapanine is hydrolyzed by myrosinase to form sulforaphane. However, during the hydrolysis of glucosinolates by plant mysosinase, nitriles, epitionitriles and cyano-epitioalkanos are formed. The proteins called ESP (epithioespecifier proteins) are responsible for the formation of theses nitriles and epitionitriles. These compounds are inactive for cancer chemoprevention or even unhealthy for humans. ESP proteins require Fe for its activity and are heat-denatured, for example when cooking vegetables rich in glucosinolates.

Generally we consume cruciferous vegetables after processing them. The myrosinase and the ESP are denatured due to the heat applied in cooking these foods which has important implications for the bioavailability of isothiocyanates. When these vegetables are consumed raw, the mysosinase from the plant itself produces a rapid hydrolysis of glucosinolates and a liberation of isothiocyanates which takes place in the upper digestive tract. In this situation the isothiocyanates are absorbed and rapidly excreted as conjugated in the urine. The consumption of these vegetables cooked using heat and the myrosinase being heat-denatured, allows the glucosinates to arrive intact to the colon, where they can undergo hydrolysis by the colonic microbiota releasing isothiocyanates in the colon itself. The sites of the digestive tract where glucosynolates are hydrolyzed and where the isothiocyanates are absorbed have implications for the protective properties against cancer that are associated with vegetables rich in glusinolates.

Some genera of the human colon microbiota (e.g. Bifidobacterium, Lactobacillus and Bacteroids) possess a myrosinase-like activity. In vitro incubation with human faeces with a food containing glucosynolates cooked to completely denature the myrosinase gives raise to the appearance of isothiocyanates. Thus, certain bacteria of the colon can hydrolyze the glucosinolates consumed in a meal with cooked vegetables rich in glucosinolates and release the hydrolysis products directly into the lumen of the colon. Due to the availability of prebiotic and probiotic supplements and the potential to express myrosinase-like activity of some bacteria of the Lactobacillus and Bifidobacterium genera, an investigation into the effect of stimulation of some bacterial populations of the colon in the metabolism of glucosinates in humans is of extraordinary interest.

That is why in the present invention the combinations of pomegranate and/or olive extracts with prebiotic effect or other prebiotics or a dietary fibre with a plant extract containing glucosinolates are described, e.g.: broccoli extract, finding that a selective stimulation of certain populations of colonic microflora, e.g. Lactobacillus and Bifidobacterium, improves the isothiocyanates bioavailability in our body formed in the colon from their precursors, the glucosinolates.

### Description of the invention

The olive polyphenols, mainly hydroxytyrosol, have shown some potential health benefits in several aspects: cardiovascular diseases prevention, degenerative diseases prevention, decreased risk of cancer, anti-inflammatory properties, reduction of the consequences caused by oxidative stress induced by smoking and skin damage protection.

Punicalagins from pomegranate have shown some potential health benefits in several aspects: cardiovascular disease prevention, degenerative disease prevention, decreased risk of cancer, anti-inflammatory, antiparasitic, antiviral and antimicrobial treatment of individuals with low amount and / or quality of sperm and treatment of erectile dysfunction.

Note that olive extracts containing hydroxytyrosol and pomegranate extracts containing punicalagins have antimicrobial activity. Surprisingly, the authors have found that standardized plant extracts for the content of these same compounds, punicalagins, have the ability to specifically promote the growth of certain beneficial bacteria in the gastrointestinal flora in humans, which had not been demonstrated until now

By the present invention has been shown that olive extract rich in hydroxytyrosol and a pomegranate extract rich in punicalagins, preferably those obtained by applications WO 2008/090460 A1 and EP 1967079 A1 (being the applicant Probelte Pharma and both are included herein by reference), respectively, acts as a prebiotic promoting the growth of beneficial bacteria of the gastrointestinal flora and inhibiting the growth of potentially pathogenic bacteria of the same.

An object of the present invention is the use as a prebiotic of a pomegranate extract containing punicalagins and free ellagic acid, in which the content of punicalagins is at least 2% (w / w) and the content of free ellagic is such that the ratio punicalagins / free ellagic acid (% w / w), is in the range of 10/1 to 35/1, and further the total phenol content is at least 5% (w / w) (expressed as gallic acid equivalent), and the solubility in water is at least 3% (w / w) (30 g pomegranate extract / L) taking into account the fact that such pomegranate extract is not a pomegranate juice as known in the prior art.

The composition of pomegranate extract above is especially useful for use as a prebiotic, due to its richness in punicalagins that can selectively stimulate the growth of colon bacteria of the genera Bifidobacterium and Lactobacillus, and the very low content of free ellagic acid present, such that the ratio punicalagins / free ellagic acid (% w / w), is in the range of 10/1 to 35/1. Several authors have reported that between the different factions that make up the pomegranate extract, free ellagic acid is one of the molecules that more inhibit microbial growth. Therefore, it is desirable that pomegranate extract to be developed for use as a prebiotic present relationship punicalagins / free ellagic acid (% w / w), as high as possible.

The commercially available best pomegranate juice contains between 2400-4000 mg / L of total polyphenols (expressed as gallic acid equivalent) including punicalagins content that is in the range 500-2000 mg / L. The said juice has a Brix of 16 and can be subsequently concentrated about 5 times thereby in punicalagins content never reaches more than 10 g / L (1% w / w). Regarding the ratio punicalagins / free ellagic acid in pomegranate juice did not exceed 8/1 and even during the usual concentration is reduced due to hydrolysis suffered by complex ellagitannins such us punicalagins with the subsequent liberation of free ellagic acid free.

Another purpose of the invention is the use of an olive extract, on its own or combined with a dietary fibre, as a prebiotic, considering that said extract is not olive oil.

More particularly, a preferred olive extract is that one obtained according to process following the WO 2008/090460 A1 application, having a content in hydroxytyrosol of at least 0.5% (w/w), and a purity of hydroxytyrosol of at least 40% (by HPLC at 280 nm). This extract is free of organic solvents (the residual content of organic solvents is less that 1 ppb and preferably from 0 ppm) and substantially free of salts, and the content in hydroxytyrosol of that extract is at least 0.5% (w/w) and the purity of hydroxytyrosol is at least 40% (by HPLC at 280 nm). A hydroxytyrosol content of at least 10% (w/w) is preferred, and more preferably at least 35% (w/w); the purity of hydroxytyrosol at 80 % is preferred, and more preferably at 90% (by HPLC at 280nm).

Another preferred pomegranate extract is that obtained according to EP application 1967079, in which the content of punicalagins is at least 5% (w / w), preferably at least 20% (w / w), and most preferably at least 30% (w / w), being the punicalagins purity of at least 55 %, and the content of free ellagic is such that the ratio punicalagins / free ellagic acid (% w / w), is in the range of 10/1 to 35/1, the total phenol content is at least 10 % (w / w), preferably at least 20% (w / w), and most preferably at least 50% (w / w) (expressed as gallic acid equivalent), and the solubility in water is at least 3% (w / w) (30 g pomegranate extract / L). Preferably, the extract has a content of residual organic solvents of least than 1 ppb and most preferably is 0 ppm..

Another preferred pomegranate extract in the present invention for use as a prebiotic is that obtained according to EP application 1967079 (where in one stage of the invention, from an extract rich in punicalagins, an extract rich in punicalins is obtained by using an enzyme capable of hydrolyzing punicalagins) characterized by being a pomegranate extract containing at least 1.5% (w / w) of punicalins, and having a ratio punicalins / free ellagic acid (% w / w) in the range of 10/1 to 35/1.

The substantial absence of even minimal traces of organic solvents such as methanol, ethanol, isopropanol that are employed in the purification steps commonly employed in the art, is of great importance for the use of the aforementioned extracts.

Additionally, some of the polyphenols present in olives are responsible for their bitterness, those being the ones that best correlate with this attribute the oleuropein and the aldehyde form of oleuropein aglicone. Thus, the use of olive extracts containing oleuropein and/or the aldehyde form of oleuropein aglicone can modify the taste of foods in which the aforementioned extract is incorporated, resulting in a lower acceptance by consumers.

Olive extract rich in hydroxytyrosol obtained according WO 2008/090460 A1 is virtually free of oleuropein and aldehyde form of oleuropein aglicone, being the addition of both components less than 5 ppm, because no organic solvent is used, where these molecules, much more hydrophobic than hydroxytyrosol are more soluble, for obtaining the extract, together with the combination of purifying steps used, chromatographic columns, for obtaining the extract.

An additional advantage of the above mentioned extracts, both pomegranate extract as olive extract, is the purification from the residues of pesticides present in raw materials, i.e. in the olives and pomegranate fruits. Thus, potentially toxic chemicals such as insecticides, herbicides, fungicides, rodenticides, etc..., such as: diuron, terbuthylazine, simazine, α-endosulfan, and β-endosulfan may be present in the starting material.

All these pesticides have molecular structures (e.g. the core of the triazines) capable of interacting with the absorption resins and then are eluted from the resin together with other components of the extract when using organic solvents. Thus the methods of preparing extracts from olives and/or pomegranate based on the use of an absorbent resin and subsequent elution with organic solvents must exercise extreme quality control on the raw material pesticide residues, since the absorption resin can act as a trap for these pesticides, concentrate on the resin and then elute them when an organic solvent is used to recover the hydroxytyrosol or the punicalagins present in the olive extracts and pomegranate extracts respectively.

The above problem was solved in the preparation of a olive extract rich in hydroxytyrosol and a pomegranate extract rich in punicalagins obtained according to the applications EP 1967079 A1, due to the fact that the non-ionic absorption resins were specifically selected to be possible to elute the active compounds of the said extract by the single use of water or an aqueous solution basified and not by the use of organic solvents.

Additionally, it is important to consider another important point. Several authors have found that certain toxic alkaloids (e.g. Pelletierine, isopeletierine and pseudopeletierine) may be present when alcohols were used to obtain extracts of pomegranate, but no alkaloids are detected when pomegranate extracts are prepared using only water as happens when the pomegranate extract rich in punicalagins is obtained according application EP 1967079 A1.

Pomegranate extract rich in punicalagins, obtained by application EP 1967079 A1, is free of alkaloids because no organic solvent in any stage of production of the extract has been used.

Specifically, the olive extract and the pomegranate extract promotes the growth of bacteria of the genera Bifidobacterium and Lactobacillus and inhibits the growth of bacteria of the genus *Clostridium* and *Bacteroides.*

Surprisingly, the pomegranate extracts and olive extracts may act at lower doses (less than 1 g of extract per day) than classical prebiotics (e.g. Inulin, FOS etc between 4-20 g day need to be effective) and even have fewer side effects (e.g., flatulence, bloating, diarrhoea, etc..), while showing a high specificity, which is an added advantage of prebiotic compositions based on these extracts.

In another scenario the present invention is shown to be possible to incorporate an olive extract rich in hydroxytyrosol and/or a pomegranate extract rich in punicalagins in a food die to produce foods that act as prebiotics to promote the growth of beneficial bacteria in the gastrointestinal flora and inhibiting the growth of bacteria potentially pathogenic thereof. As described in the relevant examples of the invention incorporating the extract did not alter the organoleptic properties of food, and punicalagins have adequate stability in different food matrices, for which in some food matrices the use of food additives commonly used as ascorbic acid and citric acid were selected.

Among the preferred food matrices highlights a refreshing fruit drink with a content of at least 12% grape juice, alone or combined with other fruit juices, containing ascorbic acid from 0 ppm to 9000 ppm to, with citric acid content between 0% and 5% and containing pomegranate extract between 50 ppm and 10000 ppm.

Due to the surprising improvement of the stability of the bioactive components of the pomegranate extract, such as punicalagins, when this was incorporated in fruit drinks where ascorbic acid and citric acid were added in appropriate proportions comparatively when not adding the above additives, was studied as influenced the addition of ascorbic acid and citric acid in the stability of the pomegranate extract obtained according to the process described in the application EP 1967079 A1 now improved by the addition of ascorbic acid (between 0.01 % and 5%) and citric acid (between 0.1 % and 10%) to the pomegranate extract in form of concentrated liquid just at the previous stage to drying in powder form by spray drying. The results are described in one of the examples below and due its exceptional interest, pomegranate extract powder containing citric acid between 0.5% and 10% (w / w) and ascorbic acid between 0.05% and 5% (p / p) are also among the objects of the present invention

Additionally, the incorporation of the extracts with prebiotic properties to the foods get a very important improvement of the oxidative stability of the same, leading the authors to claim the use of these extracts for the production of antioxidant food additives with prebiotic properties.

Another scenario of the present invention addresses the use as food additives of pomegranate extract rich in punicalagins and olive extract rich in hydroxytyrosol as an alternative to other food additives obtained by chemical synthesis and which are proving to be potentially harmful to health such as antioxidants, BHA (butylated hydroxyanisole) , BHT (butylated hydroxytoluene), , and TBHQ (*tert*-butyl hydroquinone), or preservatives such as benzoic acid, benzoates, and its derivatives, nitrates, nitrites and their derivatives, and also for the replacement of colorants such as tartrazine and erythrosine. As mentioned previously, the pomegranate extracts and olive extracts have antioxidant and antimicrobial properties, but also in the present invention is demonstrated prebiotic effect. The combination of properties is surprising it leads to be employed as food additives bioactive, so that could act on a bifunctional mode. Thus during preparation and storage of the food would take a first function as natural food additive in place of chemically synthesized additives as above mentioned, and once food is ingested their develop its function at the second level as prebiotics with consequent benefits to human health.

In another scenario the present invention is shown to be possible to incorporate an olive extract rich in hydroxytyrosol or a pomegranate extract rich in punicalagins, together with a probiotic bacteria or a mixture of probiotic bacteria in a food die to produce foods that act as symbiotic. Within these symbionts, we can find cases in which the prebiotic selectively favours the probiotic component, although the meaning of the invention is broader, referring also to the synergistic effect that can have the component prebiotic and probiotic component to achieve its beneficial effect on health. As described in the relevant examples of the invention incorporating the prebiotic and probiotic extract did not alter the stability of punicalagins when stored under appropriate conditions.

The probiotic preparation used for symbiotic is preferably a strain of Lactobacillus or Bifidobacterium. Preferably, strains are used that produce only acid L (+) lactic acid. Examples of preferred species of Lactobacillus are Lactobacillus rhamnosus, Lactobacillus casei, Lactobacillus paracasei and Lactobacillus reuteri. Particularly preferred strains are Lactobacillus rhamnosus ATCC 53103, Lactobacillus rhamnosus CGMCC 1.3724, Lactobacillus reuteri ATCC 55730. Lactobacillus paracasei CNCM 1-2116 and Lactobacillus casei DN 114-001. Examples of preferred species of Bifidobacterium are Bifidobacterium animalis, Bifidobacterium lactis, Bifidobacterium breve and Bifidobacterium longum. Particularly preferred strains are Bifidobacterium animalis DN-173 010. Bifidobacterium lactis marketed by the Danish company Christian Hansen under the trademark BB-12 and Bifidobacterium longum ATCC BAA-999 marketed by the Japanese company Morinaga Milk Industry Co. Ltd. under the trademark BB536.

Symbiotic preparation of the invention should contain between 10² and 10¹⁰ CFU of probiotic bacteria / g of symbiotic and between 0.001 % and 1% of olive extract rich in hydroxytyrosol or pomegranate extract rich in punicalagins.

The selected strain of probiotic bacteria can be cultured under any suitable method available in the prior art for its incorporation into the symbiotic preparation. Alternatively, the strain of probiotic bacteria can be purchased at a specialized supplier companies as Christian Hansen and Morinaga, so come prepared in a form suitable for addition to symbiotic.

Thus the invention relates to the use of an olive extract rich in hydroxytyrosol and/or a pomegranate extract rich in punicalagins as well as the products produced from them (food and beverages enriched with these extracts, dietary supplements, symbiotic and antioxidant food additives with prebiotic properties) to promote the beneficial effects associated with prebiotic ingredients and changes in gastrointestinal flora. These beneficial effects are the regulation of intestinal transit, modulating the immune system, mineral absorption, regulation of lipid metabolism and cancer prevention.

Another object of the present invention is the use of these new prebiotic, an olive extract rich in hydroxytyrosol and/or a pomegranate extract rich in punicalagins, and oral compositions containing these extracts in reducing risk factors associated with metabolic syndrome and its consequences, the most prominent, atherosclerosis, cardiovascular diseases, hypercholesterolemia and diabetes mellitus type 2.

Metabolic syndrome is called the set of metabolic and cardiovascular disorders that are associated with insulin resistance and abdominal obesity. One in six Europeans, and even one in three people in some EU countries, have metabolic syndrome, a disorder that greatly increases the risk of type 2 diabetes, cardiovascular disease and can cause premature death. The rapid increase in overweight and obesity at increasingly early ages explain the high prevalence of this syndrome. Among the factors of metabolic syndrome include the metabolic (obesity, type 2 diabetes, dyslipidemia, hyperglycemia) and non-metabolic (hypertension, inflammatory, prothrombotic). Metabolic syndrome is diagnosed when a person has three or more of the following characteristics:
- Abdominal obesity (waist circumference: greater than 102 cm in men or greater than 88 cm in women).
- High levels of triglycerides (greater than 150 mg / dL).
- Low levels of HDL cholesterol (less than 40 mg / dL).
- High blood pressure (greater than 130 mm Hg / 85 mm Hg).
- Fasting hyperglycemia (greater than 100 mg / dL).

In obese individuals with a family history of type 2 diabetes mellitus is more common the existence of an increased peripheral insulin resistance and postprandial hyperinsulinemia. In addition, biomarkers of inflammation are predictors of cardiovascular disease, with elevated serum C-reactive protein (CRP), interleukin 6 (IL-6), tumour necrosis factor alpha (TNF-α) and leptin, which have a higher correlation with the changes that constitute the metabolic syndrome, as well as decreased levels of adiponectin and interleukin-10 (IL-10).

There is still much to learn about the metabolic syndrome, but what we do know is that people with metabolic syndrome have an increased risk of heart attack or coronary artery disease.

In another scenario of the present invention is claimed that a pomegranate extract or an olive extract may be combined with a dietary fibre to obtain prebiotic compositions that may act synergistically in the human organism. For example, the combination of a pomegranate extract with dietary fibre not only can reduce hypercholesterolemia through the bioactivity of the compounds of the pomegranate extract, but dietary fibre (e.g., soluble fibre) to undergo fermentation in the intestine increases the production of short chain fatty acids such as propionate. Propionate is absorbed into the colon via the portal vein and has been shown to inhibit HMG-CoA reductase (HMGR).

In another scenario of the present invention is described as a combination of a pomegranate extract standardized to punicalagins with prebiotic properties with a specific mixture of probiotic bacteria (symbiotic) is useful for enhancing the production of urolithins in the lumen of the colon in humans and it is certainly of great interest in view of its application in cancer chemoprevention (the use to try to reduce the risk of cancer, retard or prevent the return).

In another scenario of the present invention the combinations of a prebiotic extract that can stimulate the growth of bacteria of the Bifidobacterium and Lactobacillus genera in the human colon with a broccoli extract where the myrosinase of the plant has previously been inactivated, were investigated. It was shown that the urinary excretion of the conjugated from the isothiocyanates present in broccoli rose after the consumption of pomegranate extract which, surprisingly, got the specific stimulation of colonic bacteria that enhance the transformation in the colon lumen of glucosinolates in the corresponding isothiocyanates which were absorbed and appeared subsequently in the urine.

In the light of the obtained results , the utility of the combinations of a vegetable source rich in glucosinolates with a source of dietary fibre is arisen, such as the soluble fibre and in a preferred scenario, one soluble fibre rich in beta-glucan obtained from oats or barley or mixtures thereof, or with a prebiotic item selected from: prebiotic oligosaccharides (fructooligosaccharides, xylooligosaccharides, polydextrose, galactooligosaccharides, manooligosaccharides, or mixtures thereof), tagatose, soybean, inulin (obtained from sources such as chicory, Jerusalem artichoke and jicama), raw oats, unrefined wheat, unrefined barley and unrefined jicama or soy or mixtures thereof.

The above combinations can be achieved, as was found in the pomegranate extract, the specific stimulation of colonic bacteria which enhance the transformation in the colon lumen of glucosinolates in the corresponding isothiocyanates with beneficial implications for human health that it entails.

Therefore, in the present invention, the combinations of olive and/or pomegranate extracts with prebiotic effect or other prebiotics or a dietary fibre with a vegetal extract containing glucosinolates, such as broccoli extract, are claimed when finding that a selective stimulation of certain populations of colonic microflora, such as Lactobacillus and Bifidobacterium, improves the bioavailability of isothiocyanates in our body; these isothocyanates are formed from their precursors, glucosinolates, in the colon.

### Description of tables and figures

Table 1 shows the sequence of the oligonucleotides used in PCR reactions in real time with SYBR Green for the quantification of bacteria in the respective samples.

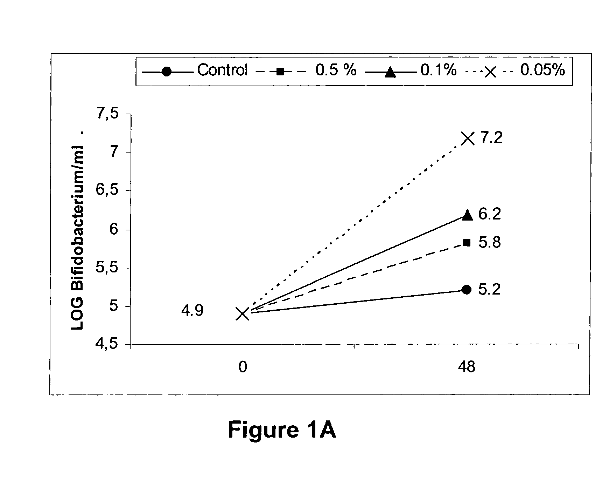

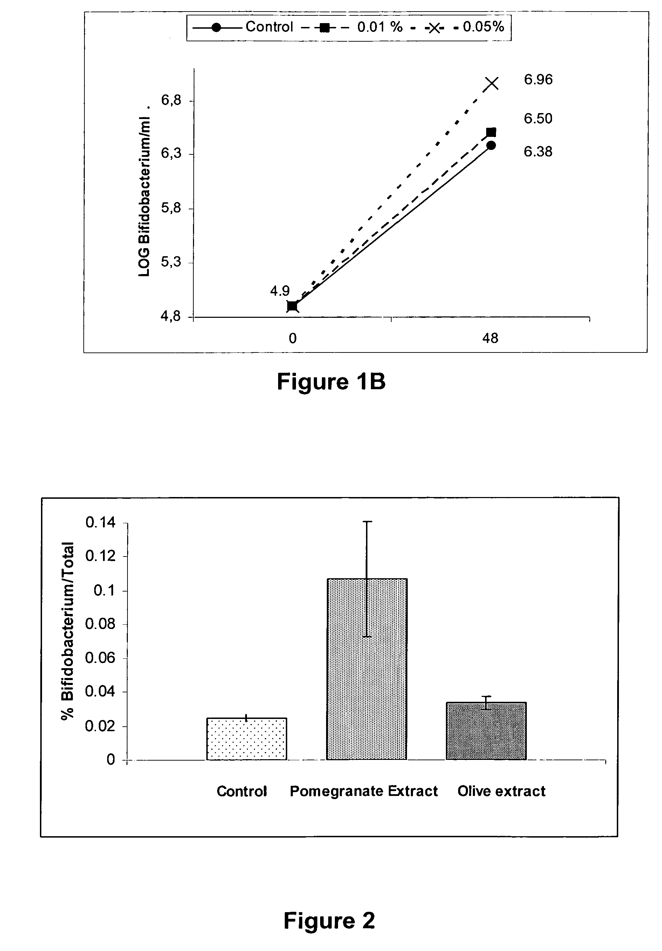

Figures 1A and 1B show the growth of bifidobacteria in the test performed and described in Examples 1 and 2 respectively. In the coordinate axis it is shown the concentration of bifidobacteria expressed as LOG10/ml. The horizontal axis shows the incubation time: 0 to 48 hours. The numbers shown in the graph correspond to the concentration of bifidobacteria, at time 0 (initial concentration of 4.9) and time 48 hours (different values for the control group, the three groups with pomegranate extract and the two groups with olive extract at different concentrations).
Figure 2 shows the results described in Example 3, the quantification of bacteria of the genus *Bifidobacterium* expressed as % with respect to the total of bacteria in the faeces of the test mice. Group 1 corresponds to the control group. Group 2 and 3 received a pomegranate extract and an olive extract respectively.
Table 2 shows the results described in Example 4 of the quantification by real-time quantitative PCR the amount of bacteria of the genus *Lactobacillus* expressed as % from the total bacteria in the stool of individuals in the trial.
Table 3 shows the results described in Example 4 of the quantification by real-time quantitative PCR the amount of bacteria of the genus Bifidobacterium expressed as% from the total bacteria in the stool of individuals in the trial.

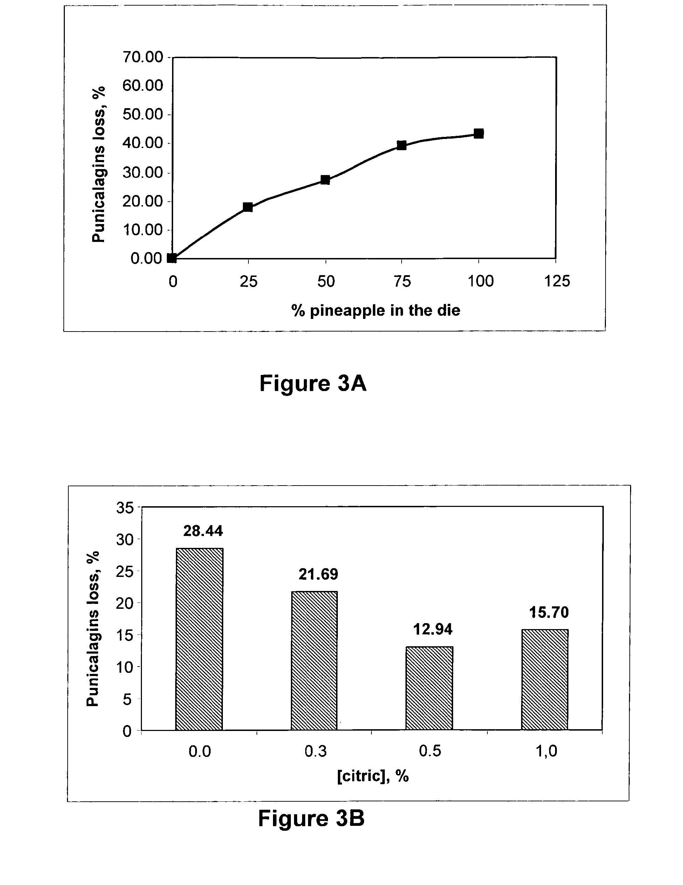

Figure 3A shows the relationship between the % fruit into a pineapple juice beverage prepared from pomegranate extract and punicalagins losses during preparation of the products.
Figure 3B shows the relationship between the% citric acid incorporated into a pineapple juice beverage (containing 50 % pineapple) prepared with pomegranate extract and punicalagins losses during preparation of the products.

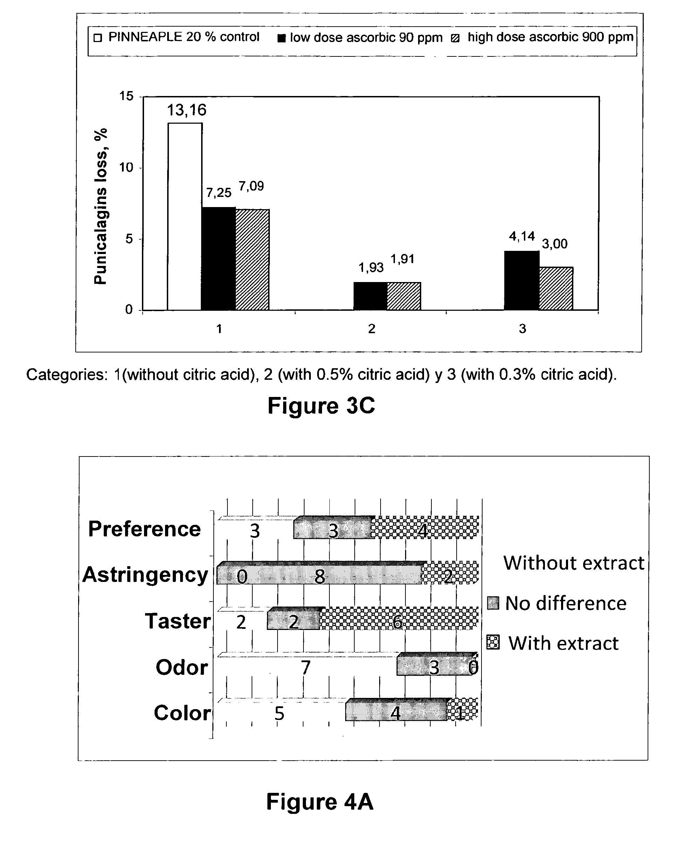

Figure 3C shows the relationship between the % ascorbic acid incorporated into a pineapple juice beverage (containing 20 % pineapple and citric acid at different concentrations) prepared with pomegranate extract and losses of punicalagins during the preparation of the products.
Table 4 summarizes the keys used for assessment by scales of the different attributes in sensory analysis of fruit juice drinks with and without pomegranate extract prepared and evaluated according to Examples 6 to 9.
   4A and 4B graphically show the results described in Example 7, the difference test and preference test, respectively, made by the assessors during the sensory analysis of the 100% apple drink.
Table 5 summarizes the evaluation of different attributes scales made by the assessors in the sensory analysis of the 100% apple drink as described in Example 7.
   5A and 5B graphically show the results described in Example 9, the difference test and preference test, respectively, made by the assessors during the sensory analysis of the Pineapple / Plum drink.
Figures 5C and 5D graphically show the results described in Example 9, the difference test and preference test, respectively, made by the assessors during the sensory analysis of the Peach / Apple drink.
Table 6 summarizes the valuation of different attributes scales made by the assessors during the sensory analysis of the Pineapple / Plum drink as described in Example 9.
Figure 6 shows the oxidative stability of the margarine samples prepared according to Example 12, as measured by the Rancimat test at 98°C and 10 l/hour. The graph shows a lineal correlation between the oxidative stability of the food matrix and the percentage of olive extract added to the food matrix.
Table 7 summarizes the evaluation of different attributes scales made by the assessors in the sensory analysis of the Peach / Apple drink as described in Example 9.
Table 8 shows the results described in Example 15 of the HPLC quantification of the total amount of isothiocyanates expressed as a percentage with respect to the total amount of isothiocyanates ingested as broccoli extract, in the 24 hours urine of the individuals in the assay.

### EXAMPLES

### EXAMPLE 1. Bifidogenic effect of an extract of pomegranate

Sterile Falcon tubes were prepared with a final volume of 50 ml containing skimmed milk, 0.4 g of a commercial yoghurt containing Bifidobacterium 10⁸ / g and a sterile solution of pomegranate extract in water to obtain the following final concentrations extract pomegranate: 0.5%, 0.1%, 0.05%, 0% (% p / V). The tubes were incubated at 30° C for 48 hours. Assays were performed in duplicate.

By real-time quantitative PCR with SYBR Green was evaluated the amount of bifidobacteria in each Falcon tube and using oligonucleotides BidF BidR (Table 1). The absolute quantification of bifidobacteria was calculated with a regression line previously obtained: y = - 0.3792 x + 17.62, where y is the Log₁₀ of the number of bifidobacteria and x is the Ct value of the quantitative PCR reaction.
The presence of pomegranate extract in the three tested concentrations promotes the growth of bifidobacteria compared to control (Figure 1A).

**Table 1**

| **Oligonucleotides** | **Sequence (5' - 3')** | **Target** |
|---|---|---|
| **AIIBF** | TCCTACGGGAGGCAGCAG | **All bacteria** |
| **AIIBR** | GGACTACCAGGGTATCTAATCCTG | **All bacteria** |
| **LacF** | TGGATGCCTTGGCACTAGGA | ***Lactobacillus* sp** |
| **LacR** | AAATCTCCGGATCAAAGCTTACTTA | ***Lactobacillus* sp** |
| **BidF** | GATTCTGGCTCAGGATGAACG | ***Bifidobacterium* sp** |
| **BidR** | GATAGGACGCGACCCCAT | ***Bifidobacterium* sp** |
| **CperF** | CGCATAACGTTGAAAGATGG | ***Clostridium perfringens*** |
| **CperR** | CCTTGGTAGGCCGTTACCC | ***Clostridium perfringens*** |
| **BacF** | ATCATGAGTTCACATGTCCG | **Bacteroides sp** |
| **BacR** | CCTGCCTCTACTGTACTC | ***Bacteroides* sp** |

### EXAMPLE 2. Bifidogenic effect of an olive extract

Sterile Falcon tubes were prepared with a final volume of 50 ml containing skimmed milk, 0.4 g of commercial yoghourt containing 10⁸ bifidobacterium/gr and a sterile solution of olive extract in water to obtain the following final concentrations of olive extract: 0.05%; 0.01 %; 0% (% w/V). The tubes were incubated at 30°C for 48 hours. Assays were performed in duplicate.

The amount of bifidobacteria in each Falcon tube was evaluated by real-time quantitative PCR with SYBR Green and using oligonucleotides BidF and BidR (Table 1). The absolute quantification of bifidobacteria was calculated with a regression line previously obtained: y = - 0.3792x + 17, 62, where **y** is the Log₁₀ of the quantity of bifidobacteria and **x** is the Ct value of the quantitative PCR reaction.

The presence of olive extract in the two tested concentrations promotes the growth of bifidobacteria with respect to control (Figure 1 B).

### EXAMPLE 3. Prebiotic effect of pomegranate extract in an animal model

The trial was conducted with three groups of 10 mice each. The mice were fed for 20 days with commercial feed and a daily dose of 12.8 mg of pomegranate extract per mouse (equivalent to a daily intake of 320 mg pomegranate extract / kg body mass) (group 2) or a daily dose of 0.9 mg of olive extract per mouse (equivalent to a daily intake of 22.9 mg olive extract / kg body mass) (group 3) or without pomegranate extract (group 1, control). After this period, the faeces of each group were collected for bacterial DNA isolation from 0.2 g of the same.

Using quantitative real-time PCR was assessed the amount of beneficial bacteria in the faeces, expressed as follows: % bacteria of the genus Bifidobacterium / total bacteria. Oligonucleotides were used BidF and BidR (Table 1). The relative quantification was performed using the method described by Liu and Saint, 2002, making each measurement in triplicate.

Here are the results that show a clear effect on the administration of pomegranate extract and olive extract to promote the growth of bacteria of the genus *Bifidobacterium.* The graphical representation of the results is shown in Figure 2.

| | *% Bifidobacterium* /Total |
|---|---|
| Group 1 (control) | 0.0248 ± 0.0021 |
| Group 2 (pomegranate extract) | 0.1069 ± 0.0341 |
| Group 3 (olive extract) | 0.0336 ± 0.0038 |

In group 2, there is a significant difference (p <0.05) compared to group 1 (control).

### EXAMPLE 4. Prebiotic effect of pomegranate extract on a human intervention study: changes in gastrointestinal flora.

The assay was performed with 6 healthy individuals aged between 28 and 53 years. Each individual took a daily capsule containing 575 mg of pomegranate extract to lunch. They were told the volunteers to avoid eating prebiotic capacity products (such as fructooligosaccharides, inulin, etc.), antibiotics, laxatives, and especially products with prebiotics (i.e. unpasteurized yogurt, fermented milk drinks, etc). The 6 volunteers began to follow these restrictions in their diet from 14 days pre-treatment phase of the intervention study, continuing the same during the 28 days of treatment, where they were to consume one capsule with pomegranate extract daily.

Faecal samples were taken from each of the individuals at time 0 (after the 14 days prior to the administration of pomegranate extract) and after 28 days of administration. From the stool was performed isolation of bacterial DNA.

Using quantitative real-time PCR was assessed the amount of beneficial bacteria in the faeces, expressed as follows: % Lactobacillus bacteria / total bacteria or bacteria of the genus Bifidobacterium % / total bacteria. LacF oligonucleotides were used, LACR, BidF and BIDR (Table 1). Furthermore, we assessed the amount of potentially pathogenic bacteria in the stool, expressed as follows: % bacteria of the species Clostridium perfringens / total bacteria or bacteria of the genus Bacteroides% / total bacteria. CperF oligonucleotides were used, CperR, BacF and BacR (Table 1). The relative quantification was performed using the method described by Liu and Saint, 2002, making each measurement in triplicate.

The results are shown below:

### A) Beneficial bacteria:

*Lactobacillus:* 5 out of 6 individuals (83.3%) had a significant increase (p< 0.05 ó p< 0.01 depending on the case) in the percentage of bacteria of this genus after 28 days of administration of pomegranate extract.

**Table 2**

| Patient n° | *Lactobacillus* t = 0 | *Lactobacillus* t = 28 | Significant difference |
|---|---|---|---|
| **1** | **0.0287 ± 0.0063** | **0.1741 ± 0.0899** | **p < 0.05** |
| **2** | **0.0716 ± 0.0398** | **0.5755 ± 0.0691** | **p < 0.001** |
| **3** | **0.2130 ± 0.0572** | **0.0816 ± 0.0059** | - |
| **4** | **0.0576 ± 0.0185** | **0.1443 ± 0.0347** | **p < 0.05** |
| **5** | **0.0635 ± 0.0185** | **0.2095 ± 0.0777** | **p < 0.05** |
| **6** | **0.0127 ± 0.0044** | **0.2019 ± 0.0082** | **p < 0.001** |

*Bifidobacterium:* 5 out of 6 individuals (83, 3%) had a significant increase (p< 0. 05 ó p< 0. 01 depending on the case) in the percentage of bacteria of this genus after 28 days of administration of pomegranate extract.

**Table 3**

| Patient n° | *Bifidobacterium* t = 0 | *Bifidobacterium* t = 28 | Significant Difference |
|---|---|---|---|
| **1** | **0.2191 ± 0.0355** | **0.1522 ± 0.0116** | **-** |
| **2** | **0.1043 ± 0.0066** | **0.4718 ± 0.0177** | **p < 0.001** |
| **3** | **0.3004 ± 0.0513** | **0.4459 ± 0.0342** | **p < 0.05** |
| **4** | **0.4196 ± 0.0256** | **1.1832 ± 0.1177** | **p < 0.001** |
| **5** | **0.1060 ± 0.0044** | **0.2317 ± 0.0504** | **p < 0.05** |
| **6** | **0.8991 ± 0.0461** | **1.2721 ± 0.0576** | **p < 0.001** |

### B) Pathogenic bacteria:

*Clostridium perfringens:* 5 out of 6 individuals (83, 3%) showed a significant decrease (p< 0. 05 ó p< 0. 01 depending on the case) in the percentage of bacteria of this genus after 28 days of administration of pomegranate extract.

*Bacteroides:* 4 out of 6 individuals (66, 7%) showed a significant decrease (p< 0. 05 ó p< 0. 01 depending on the case) in the percentage of bacteria of this genus after 28 days of administration of pomegranate extract.

### EXAMPLE 5. Prebiotic effect of pomegranate extract on a human model: regulation of intestinal transit

During the test at 6 individuals described in Example 4 were interviewed each of them about their digestive health during the administration of pomegranate extract.

All individuals (100%) reported not having had any digestive problems during treatment.

5 individuals (83.3%) reported having achieved regularity unusual in them in the intestinal transit. The other individual said to have observed no changes in their habits, which themselves were already scheduled.

### EXAMPLE 6. Preparation of juice or fruit nectar prebiotic with an extract of pomegranate, evaluation of the stability of the pomegranate extract in the food die after preparation and subsequent storage.

A sample of pomegranate extract with a content of 61.12% punicalagins (w / w) obtained according to EP 1967079 A1 application was used to prepare pineapple and apple juice and peach nectar containing the extract above mentioned at concentration of 624 mg of pomegranate extract / kg of food die (381.4 mg punicalagins / kg of food die). The raw materials used were: pineapple juice concentrate with 60 ° BRIX, apple juice concentrate with 70 ° BRIX and peach puree. The prototypes, control (without extract) and prebiotic (with 624 ppm of pomegranate extract) of the three food matrices with and without pomegranate extract were prepared according to the following recipes:

| **Pinneaple juice** | | |
|---|---|---|
| **Description** | **g / Kg** | **%** |
| **PINEAPPLE CONC. 60° BX.** | 197.89 | 19.79 |
| **WATER** | 802.11 | 80.21 |
| **% Fruit** | 100.0 % | |
| **°Brix** | 12.8 % | |
| **Density** | 1.05174 g/ ml | |
| **Acidity** | 0.600 g c.a./100 ml | |

With and Without Pomegranate Extract.

With Extract: 624 mg./l.

| | **° Bx.** | **pH/t^{a}** | **Acidity (g.c.a/100ml)** |
|---|---|---|---|
| **Without extract** | 12.8 | 3.66/27.2 | 0.592 |
| **With extract** | 12.8 | 3.68/24.6 | 0.610 |

| **APPLE JUICE** | | **05.11.08** |
|---|---|---|
| **Description** | **g/Kg** | **%** |
| **APPLE CONC. 70° BX.** | 161.04 | 16.10 |
| **WATER** | 838.96 | 83.89 |
| **% Fruit** | 100.0 % | |
| **°Brix** | 11.2 % | |
| **Density** | 1.04502 g/ ml | |
| **Acidity** | 0.350 g c.a./100 ml | |

| | **° Bx.** | **pH/t^{a}** | **Acidity (g.c.a./100ml)** |
|---|---|---|---|
| **Without extract** | **11.4** | **3.72/19.5** | **0.304** |
| **With extract (624mg/l)** | **11.5** | **3.73/18.4** | **0.31** |

| **Peach nectar** | | |
|---|---|---|
| **Description** | **g/Kg** | **%** |
| **PEACH PUREE** | 404.6 | 40.46 |
| **CITRIC ACID** | 2.0 | 0.20 |
| **SUGAR** | 62.2 | 6.22 |
| **WATER** | 576.2 | 57.60 |
| **% Fruit** | 55.0 % | |
| **°Brix** | 11.2 % | |
| **Density** | 1.04502 g/ ml | |
| **Acidity** | 0.480 g.c.a./ 100 ml | |

| | **° Bx.** | **pH/t^{a}** | **Acidity (g.c.a./100ml)** |
|---|---|---|---|
| **Without extract** | 11.3 | 3.52/23.4 | 0.467 |
| **With extract (624mg./l.)** | 11.4 | 3.53/25.0 | 0.477 |

The 6 prototypes were pasteurized at 100° C for 15 seconds and aseptically packaged in glass containers at a temperature of 85 to 87 ° C. The 6 glass jars containing the prototypes were immersed in water to cool quickly and then, losses of pomegranate extract incorporated into the 3 types of juices / nectars was measured by determining the punicalagins content by HPLC method.

Results are shown below:

| Juice/nectar with pomegr. extr. | Pomegr. Extr. losses,% |
|---|---|
| Pineapple juice | 43.2% (156 mg punicalagins) |
| Apple juice | 1.1% (4.3 mg punicalagins) |
| Peach nectar | 39.8% (152 mg punicalagins) |

The first conclusion is that pomegranate extract has stability dependent on the food die. Only apple juice showed an ideal behaviour for the application of prebiotic pomegranate extract. In the case of apple juice, when the pomegranate extract is added, it cannot be called juice, since the law only allows the addition of vitamins and minerals to juices, therefore even containing 100% fruit it will be referred to as Apple Drink.

In order to prepare pineapple and peach drinks with Pomegranate extract a study of factors influencing the losses of the pomegranate extract in these food matrices is carried out, finding 3 important factors: % fruit, % citric and [ascorbic acid] (see Figures 3A, 3B and 3C referred to the pineapple drink; the data for the peach drink were not shown but were on the same line.

After surprisingly discovering that the stable incorporation of pomegranate extract to food matrices of the juice / nectar type seemed enhanced by the inclusion in the formulation of citric acid, ascorbic acid supplementation and the presence of a base of apple juice, we proceeded to prepare a new type of multifruit nectar with pomegranate extract.

The prototypes, control (without extract) and prebiotic (with 624 ppm of pomegranate extract) multifruit nectar with and without pomegranate extract were prepared according to the following recipe:

| **MULTIFRUIT NECTAR** | |
|---|---|
| **Description** | **%** |
| **APPLE JUICE** | 30.0% |
| **GRAPE JUICE** | 18.0% |
| **KIWI JUICE** | 2.0% |
| **PRUNE JUICE** | 7.0% |
| **ASCORBIC ACID** | 0.090% |
| **CITRIC ACID** | 0.50% |
| **POMEGRANATE EXTRACT** | 0.0624% |
| **SUGAR** | 0.47% |
| **WATER** | 42.40% |
| **% Fruit** | 57.0 % |
| **°Brix** | 11.2 % |
| **Density** | 1.04502 g/ml |
| **Acidity** | 0.580 g.c.a./100 ml |

The 2 prototypes were pasteurized at 100 ° C for 15 seconds and aseptically packaged in glass containers at a temperature of 85 to 87 ° C. The 2 glass jars containing the prototypes were immersed in water to cool quickly and then, losses of pomegranate extract on nectar multifruit incorporated was measured by determining the punicalagins content by HPLC method.

Results are shown below:

| Juice/nectar with pomegr. extr. | Pomegr. Extr. losses,% |
|---|---|
| Multifruit nectar | 1.5% (5.7 mg punicalagins) |

The main conclusions are that it is possible to stably incorporate pomegranate extract with prebiotic properties in food matrices of the type of juice / nectars (containing 100% fruit, and at least 50% fruit respectively) The most optimal die is apple juice, followed by grape juice but also dies with various fruits conveniently stabilized through the incorporation of between 90 and 900 ppm ascorbic acid and between 0.3 and 0.5 of citric acid exhibit excellent incorporation of pomegranate extract.

Additionally, the bottles with prototypes of apple drink and nectar multifruit were stored to 5 ± 3 ° C and 25 ± 2 ° C. The losses of pomegranate extract incorporated into the apple drink and nectar multifruit were analyzed by determining the content punicalagins by HPLC method. After 6 months of stability testing punicalagins loss was less than 5% in the two conditions under test:
a) 5±3 °C.
b) 25±2 °C with a humidity of 60 ±5 RH

### EXAMPLE 7. Sensory analysis of juice or fruit nectar prebiotic with pomegranate extract.

We proceeded to **Sensory Analysis** of apple juice and nectar multifruit with and without pomegranate extract prepared according to Example 6. We conducted a tasting session for flavour, namely the realization of a sensory evaluation designed by the panel of expert judges, in order to identify the characteristics and / or product attributes with and without the addition of pomegranate extract.
- The tasting sessions consist of a panel of tasters 10-12 semi-trained in the products.
- The tastings included:
   **Design of a questionnaire** comprising the most significant attributes of appearance, flavour, aroma and texture, and overall assessment. The questionnaire was approved by expert tasters.

### Characterization

It was conducted by paired comparison of samples containing pomegranate extract and samples that did not contain.

In this questionnaire the following tests were assessed:
✔ Proof of difference, in which the tasters were asked if they detect any difference between the samples submitted with respect to different attributes.
✔ Preference test, in which the tasters were asked which of the samples they liked best.
✔ Proof of intensity, in which the tasters were asked which of the samples, had greater intensity in different attributes.
✔ Proof of valuation scales of the different attributes (colour, aroma, sweetness /acidity, flavour, astringency and General Rating).
✔ Finally, we have carried out the statistical analysis and drafted a report with the results summarized below.

The keys to the valuations of the attributes are summarized in the following table:

**Table 4**

| **Colour/Aroma/Taste** | **Sweetness-Acidity:** | **Astringency:** |
|---|---|---|
| 1. Extremely pleasant | 1. Extremely sweet | 1. Extremely astringent |
| 2. Very pleasant | 2. Very sweet | 2. Very astringent |
| 3. Moderately pleasant | 3. Moderately sweet | 3. Moderately astringent |
| 4. Slightly pleasant | 4. Slightly sweet | 4. Slightly astringent |
| 5. Slightly unpleasant | 5. Slightly acidic | 5. Not astringent |
| 6. Moderately unpleasant | 6. Moderately acidic | |
| 7. Very unpleasant | 7. very acidic | |
| 8. Extremely unpleasant | 8. Extremely acidic | |

| **Overall rating** | | |
|---|---|---|
| 1. I love it | | |
| 2. I like it very much | | |
| 3. I like it slightly | | |
| 4. I neither like nor dislike it | | |
| 5. I dislike it slightly | | |
| 6. I dislike it very much | | |
| 7. I hate it | | |

### Sensory analysis of the apple drink.

In the difference test, a greater number of tasters as compared with other products could not find appreciable differences between the samples studied for attributes given.

Regarding the *Preference* there is not a general Choice for any of the two samples.

The graphical representation of the results is shown in Figure 4A.

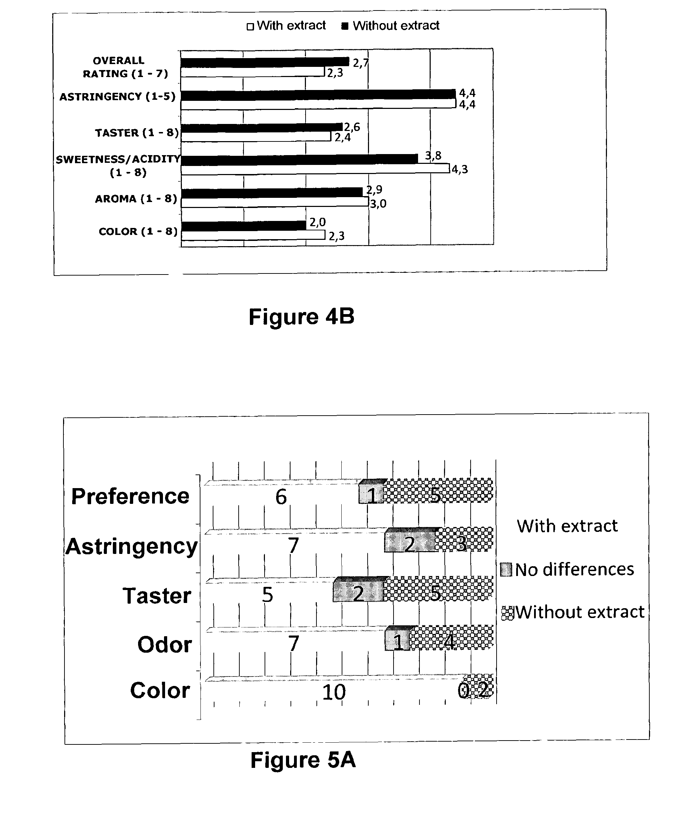

The results of the preference test characterization of the apple drink with and without extract are shown in Figure 4B.

Regarding the valuation by scales of different attributes, significant differences (p ≤ 0.05) in the *colour* characteristics were found.

**Table 5**

| | | **SAMPLE** | |
|---|---|---|---|
| **ATTRIBUTE** | **SCALE** | **With extract** | **Without extract** |
| **COLOUR** | 1- extremely pleasant | 2,3 | 2,0 |
| | 8- extremely unpleasant | | |
| **AROMA** | 1- extremely nice | 3,0 | 2,9 |
| | 8- extremely unpleasant | | |
| **SWEETNESS/ACIDITY** | 1- extremely sweet | 4,3 | 3,8 |
| | 8- extremely acidic | | |
| **TASTER** | 1- extremely nice | 2,4 | 2,6 |
| | 8- extremely unpleasant | | |
| **ASTRINGENCY** | 1- extremely astringent | 4,4 | 4,4 |
| | 8- not astringent | | |
| **OVERALL RATING** | 1- love it | 2,3 | 2,7 |
| | 8- I dislike it very much | | |

Based on these results, we can say that the apple drink containing pomegranate extract is valued positively from the sensory point of view, even more favourably than the Apple drink without extract.

### Sensory Analysis of the Multifruit Drink.

Assessment Outcome:
- Overall rating: The product was rated between "I love it " and " I like it slightly".
- Astringency: *"slightly astringent".*
- Taste: between *"highly and moderately pleasant".*
- Sweetness/acidity: *"moderately sweet".*
- Aroma: *"very pleasant".*
- Colour: between *"highly and moderately pleasant".*

The addition of pomegranate extract on drink of apple, grape, kiwi and plum provides no unpleasant colours, aromas or flavours.

Based on these results, we can say that the multifruit drink containing pomegranate extract is valued positively from the sensory point of view.

### EXAMPLE 8. Preparation of a prebiotic fruit drink / drink tea extract with an extract of pomegranate, evaluation of the stability of the pomegranate extract in the food matrix.

A sample of pomegranate extract with a content of 61.12% punicalagins (w / w) obtained according to EP 1967079 A1 application was used to prepare fruit drinks with or without tea extracts containing pomegranate extract concentration 624 mg of ex. Pomegranate / kg of food die (381.4 mg punicalagins / kg of food matrix). The prototype control (without extract) and prebiotic (with 624 ppm of pomegranate extract) of various food matrices with and without pomegranate extract were prepared according to the following recipes:

| **PINEAPPLE AND PLUM DRINK** | | |
|---|---|---|
| **Description** | **g/Kg** | **%** |
| **PINEAPPLE CONC.** | 18.6 | 1.86% |
| **PLUM CONC.** | 14.3 | 1.43% |
| **CITRIC ACID** | 5.00 | 0.50% |
| **ASCORBIC ACID** | 0.90 | 0.09% |
| **POMEGRANATE EXTRACT** | 0.624 | 0.0624% |
| **SUGAR** | 80.20 | 8.2% |
| **WATER** | 880.40 | 88.0% |
| **% Fruit** | 20.0 % | |
| **°Brix** | 11.20 % | |
| **Density** | 1.045021 g/ ml | |
| **Acidity** | 0.555 g c.a./ 100 ml | |

| **PEACH AND APPLE DRINK** | | |
|---|---|---|
| **Description** | **g / Kg** | **%** |
| **PEACH PURE** | 73.1 | 7.30 |
| **APPLE CONC.** | 16.3 | 1.60 |
| **CITRIC ACID** | 5.0 | 0.50 |
| **ASCORBIC ACID** | 0.9 | 0.09 |
| **SUGAR** | 90.2 | 9.02 |
| **POMEGRANATE EXTRACT** | 0.624 | 0.0624 |
| **WATER** | 814.4 | 81.40 |
| **% Fruit** | **20.3 %** | |
| **° Brix** | 1**1.2 %** | |
| **Density** | **1.04502 g/ ml** | |
| **Acidity** | **0.550 g c.a./100 m**l | |

| **Apple, cranberry and red tea drink** | | |
|---|---|---|
| Description | g / Kg | % |
| | | |
| APPLE CONC. | 61.94 | 6.2 |
| CRANBERRY CONC. | 2.86 | 0.29 |
| CITRIC ACID | 5 | 0.5 |
| ASCORBIC ACID | 0.9 | 0.09 |
| TEA EXTRACT PU ERH (RED) | 1.2 | 0.12 |
| SUGAR | 90.2 | 9.02 |
| POMEGRANATE EXTRACT | 0.624 | 0.0624 |
| WATER | 837.3 | 81.4 |
| % Fruit (38 % APPLE + 2 % CRANBERRY) | 40 % | |
| °Brix | 11.1 % | |
| Density | 1.04525g /ml | |
| Acidity | 0.56 q c.a./100 ml | |

| **Grape, lemon and black tea drink** | | |
|---|---|---|
| Description | g / Kg | % |
| GRAPE CONC. | 61.94 | 6.2 |
| LEMON CONC. | 3.45 | 0.34 |
| CITRIC ACID | 5 | 0.5 |
| ASCORBIC ACID | 0.9 | 0.09 |
| BLACK TE EXTRACT | 2 | 0.2 |
| SUGAR | 90.2 | 9.02 |
| POMEGRANATE EXTRACT | 0.624 | 0.0624 |
| WATER | 836 | 81.4 |
| % Fruit (38 % GRAPE + 2 % LEMON) | 40 % | |
| °Brix | 11.2% | |
| Density | 1.04452g /ml | |
| Acidity | 0.55q a.c.a./100 ml | |

The 8 prototypes were pasteurized at 100° C for 15 seconds and aseptically packaged in glass containers at a temperature of 85 to 87 ° C. The 8 glass containers containing the prototypes were immersed in water to cool quickly and then, losses of pomegranate extract incorporated into fruit drinks were measured by determining the content in punicalagins by HPLC method.

The results are shown below:

| Fruit drink with Pomegr. Extr. | Loss of pomegranate extr.,% |
|---|---|
| Pineapple/Plum drink | 0% (0 mg punicalagins) |
| Peach/Apple drink | 7.5% (28.6 mg punicalagins) |
| Fruit drink and red tea extract | 0% (0 mg punicalagins) |
| Fruit drink and black tea extract | 0% (0 mg punicalagins) |

The principal conclusions are that it is possible to stably incorporate pomegranate extract with prebiotic properties in food matrices of the type of fruit drinks with or without tea extracts (which contain from 5 to 50% fruit and from 0.01 to 1% of red, black, white, green, tea extract) The most optimal matrices containing as the main basis apple juice and / or grape juice and the addition of between 90 and 900 ppm of ascorbic and between 0.3 and 0.5 citric acid provides excellent stability with the addition of pomegranate extract.

Additionally bottles with peach/apple drink and pineapple/plum drink were stored at 5 ± 3°C and 25 ± 2°C. The losses of pomegranate extract incorporated in both fruit drinks were analyzed by determining the punicalagins content by HPLC method. After 6 months of stability testing punicalagins loss was less than 5% in both conditions under test:
a) 5±3 °C.
b) 25±2 °C with a humidity of 60 ±5 RH

### EXAMPLE 9. Sensory analysis of a prebiotic fruit drink / tea extract with an extract of pomegranate.

We proceeded to the Sensory Analysis, according to that described in Example 7, regarding the pineapple and plum drink and regarding the peach and apple drink, with and without pomegranate extract prepared according to Example 8.

### Sensory analysis of the pineapple and plum drink.

In the Proof of Difference, 100% of the panellists identified a difference between the samples. These differences are significant (p ≤ 0.05) only for the colour parameter, where 10 of the 12 tasters identified as darker the sample which contained the pomegranate extract. However, the sample with the extract is preferred by 50% of the tasters.

The graphical representation of the results is shown in Figure 5A.

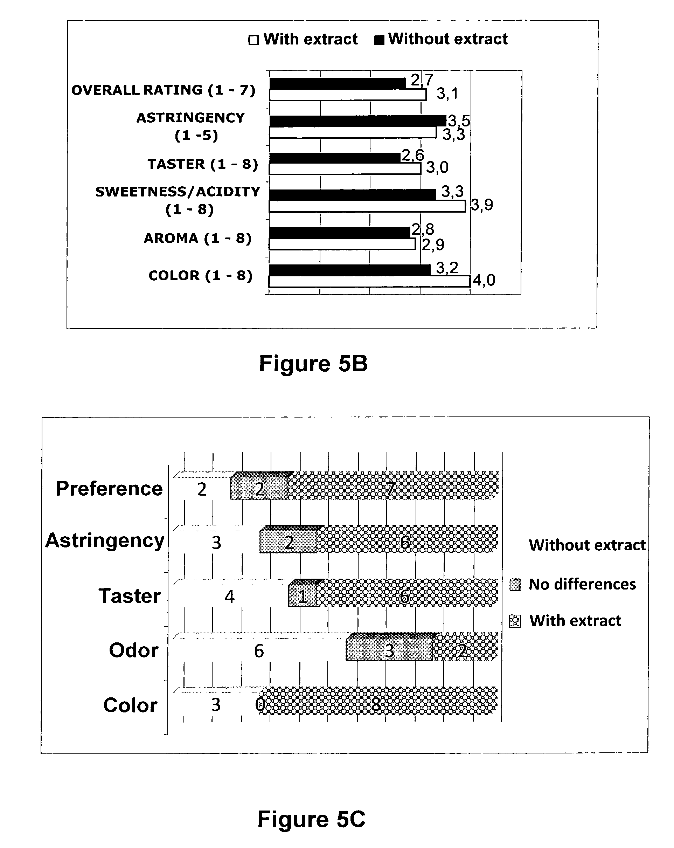

The results of the characterization preference test of the pineapple/plum drinks with and without extract are shown in Figure 5B.

As for the valuation by scales of the different attributes, the results are similar to those obtained in the Proof of Difference and Proof of Intensity.

There are significant differences (p≤0.05) in the colour and sweetness/acidity characteristics.

**Table 6**

| | | **SAMPLE** | |
|---|---|---|---|
| **ATTRIBUTE** | **SCALE** | **With extract** | **Without extract** |
| **COLOR** | 1- extremely pleasant | 4,0 | 3,2 |
| | 8- extremely unpleasant | | |
| **AROMA** | 1- extremely pleasant | 2,9 | 2,8 |
| | 8- extremely unpleasant | | |
| **SWEETNESS/ACIDITY** | 1- extremely sweet | 3,9 | 3,3 |
| | 8- extremely acidic | | |
| **TASTER** | 1- extremely pleasant | 3,0 | 2,6 |
| | 8- extremely unpleasant | | |
| **ASTRINGENCY** | 1- extremely astringent | 3,3 | 3,5 |
| | 8- not astringent | | |
| **OVERALL RATING** | 1- I love it | 3,1 | 2,7 |
| | 8- I dislike it very much | | |

Based on these results, we conclude that the pineapple and plum drink containing pomegranate extract has been evaluated in all attributes analyzed, positively from the sensory point of view

### Sensory Analysis of the Peach/Apple Drink.

In the Proof of Difference, 100% of the panellists identified a difference between the samples. These differences are significant (p ≤ 0.05) for the parameters of *Colour,* Flavour and Astringency.

However, the sample containing pomegranate extract is preferred by 7 of the 11 tasters.

The graphical representation of the results is shown in Figure 5C.

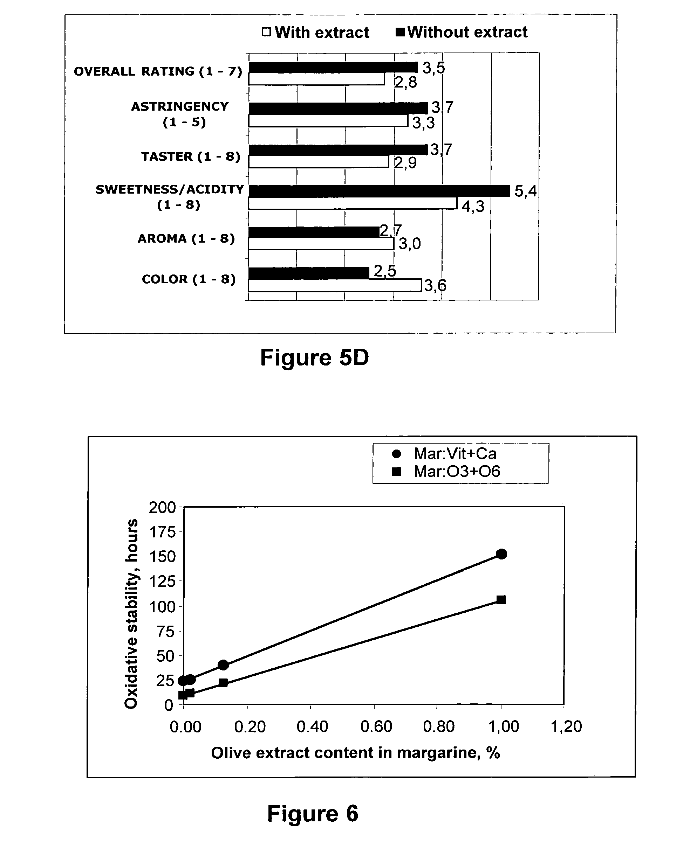

The results of the preference test characterization of the Peach / Apple drink with and without extract are shown in Figure 5D.

As for the evaluation by the different attributes scales, the results are similar to those obtained in the difference / intensity tests.

There are significant differences (p ≤ 0.05) in the colour, flavour and astringency characteristics.

**Table 7**

| | | **SAMPLE** | |
|---|---|---|---|
| **ATTRIBUTE** | **SCALE** | **With extract** | **Without extract** |
| **COLOR** | 1- extremely pleasant | 3,6 | 2,5 |
| | 8- extremely unpleasant | | |
| **AROMA** | 1- extremely pleasant | 3,0 | 2,7 |
| | 8- extremely unpleasant | | |
| **SWEETNESS/ACIDITY** | 1- extremely sweet | 4,3 | 5,4 |
| | 8- extremely acidic | | |
| **TASTER** | 1- extremely pleasant | 2,9 | 3,7 |
| | 8- extremely unpleasant | | |
| **ASTRINGENCY** | 1- extremely astringent | 3,3 | 3,7 |
| | 8- not astringent | | |
| **OVERALL RATING** | 1- I love it | 2,8 | 3,5 |
| | 8- I dislike it very much | | |

Based on these results, we conclude that the peach and apple drink containing pomegranate extract, even showing a slight astringency, is valued positively from the sensory point of view.

### EXAMPLE 10. Preparation of a symbiotic fruit drink with a strain of probiotic bacteria and a pomegranate extract, evaluation of the stability of the probiotic in the food die.

A sample of pomegranate extract with a content of 61.12% punicalagins (w / w) obtained according to EP 1967079 A1 application was used to prepare fruit drinks with pomegranate extract at a concentration of 624 mg pomegranate extract / kg of food die (381.4 mg punicalagins / kg of food die). The fruit drink with pomegranate extract was prepared according to the following recipe:

| **Fruit drink with pomegranate extract** | | |
|---|---|---|
| Description | g / Kg | % |
| APPLE CONC. | 61.94 | 6.2 |
| GRAPE CONC. | 3.26 | 0.33 |
| CITRIC ACID | 5 | 0.5 |
| ASCORBIC ACID | 0.9 | 0.09 |
| SUGAR | 90.2 | 9.02 |
| POMEGRANATE EXTRACT | 0.624 | 0.0624 |
| WATER | 838.1 | 83.8 |
| % Fruit (38 % APPLE + 2 % GRAPE) | 40 % | |
| °Brix | 11.1 % | |
| Density | 1.04525g /ml | |
| Acidity | 0.56 q. c.a../100 ml | |

The fruit drink with pomegranate extract was pasteurized at 100°C for 15 seconds and aseptically packaged in glass containers at a temperature of 85 to 87°C. Glass containers containing the prototypes of fruit drink with pomegranate extract were immersed in water to cool quickly. After reaching 25°C, the bottles with the drink were moved to a laminar flow hood where in a completely aseptic way a lyophilized product, the probiotic bacteria Bifidobacterium lactis marketed by the Danish company Christian Hansen under the trademark BB-12 was added.

The bottles were re-sealed, stirred manually for the dissolution of the lyophilized powder of the probiotic product and stored in a cold chamber at a temperature of 5 ± 3°C. The content in CFU / g of beverage was determined by plate count method at 0 and 30 days from the start of storage.

The results are shown below:

| | |
|---|---|
| 0 days of storage | 4x10⁸ CFU/ g drink |
| 30 days of storage | 9x10⁷ CFU/ g drink |

The main conclusions are that it is possible to stably incorporate pomegranate extract with prebiotic properties together with a strain of probiotic bacteria in food matrices of the type of fruit drinks containing from 5 to 50% fruit and from 0 to 9000 ppm of ascorbic acid and from 0 to 5% citric acid. The content of live probiotic bacteria after 1 month's storage symbiotic fruit drink was over 10⁷ CFU / g of symbiotic drink.

Thus the invention contemplates a food comprising a beverage containing at least 10% apple juice, combined or not with other fruit juices, with an ascorbic acid content from 0 to ppm to 9000 ppm, with citric acid content between 0% and 5%, with a content of probiotic bacteria in a range between 10² to 10¹⁰ CFU / g of composition and containing pomegranate extract or olive extract, between 50 ppm and 10000 ppm.

### EXAMPLE 11. Preparations of pomegranate extract powder with improved stability by the addition of ascorbic acid and citric acid.

Due to the surprising improvement of the stability of the bioactive components of the pomegranate extract, such as punicalagins, when this was incorporated in fruit drinks where ascorbic acid and citric acid were added in appropriate proportions comparatively when not adding the above additives (Figures 3B and 3C), was studied as influenced the addition of ascorbic acid and citric acid in the stability of the pomegranate extract.

Two samples of 10 L of the pomegranate extract in form of concentrated liquid just at the previous stage to drying in powder form by spray drying obtained according to Example 7 of the application EP 1967079 A1, were separated. 90 ppm ascorbic acid and 0.5% citric acid were added to a sample of 10 L, and stirred to dissolve completely. The other sample of 10 L acts as a control and ascorbic acid or citric acid were not added. Then, two samples of pomegranate extract in form of dry powder were obtained following the drying procedure described in Example 8 of application EP 1967079 A1. First the control sample without additives was dried (sublot 1) and then after cleaning the spray drier, the sample with additives was dried (sublot 2). The two sublots of pomegranate extract are packaged in heat sealed aluminium foil bags containing 10 g per bag and 5 samples from each sublot. All bags were placed in an incubation climatic chamber to carry out an accelerated stability study at a temperature of 70 ± 2°C and humidity 75 ± 5%.

The stability of pomegranate extract on samples of the sublot 1 and 2 was measured at different times by determining the punicalagins content by HPLC method.
After 30 days of incubation under the conditions mentioned punicalagins loss was less than 5% for sublot 2 (sample with additives), while for the sublot 1 (control without additives) punicalagins loss was over 15%:

### EXAMPLE 12. Preparation of a prebiotic margarine with olive extract substituting the Chemicals antioxidant tert-butylhydroquinone, evaluation of its oxidative stability and hydroxytyrosol's stability.

A sample of olive extract containing 23.57% (w/w) of hydroxytyrosol, obtained according to the application WO 2008/090460 was used to make margarines containing the above mentioned extract at different concentrations, namely at 0. 0.02, 0.13 and 1.00%. 8 margarine samples were prepared, each one of them weighing around 100 g. 4 samples of one margarine contained the usual ingredients and were enriched with vitamins A, D and E and calcium (Mar: Vit+Ca) and the other 4 samples of another margarine contained the usual ingredients and were enriched with omega fatty acids 3 and 6 (Mar: O3 + O6). The 8 samples were melted using Bain Marie at 55°C and the desired amount of olive extract was added, except for the two control samples where instead of olive extract the legally permitted dose of the synthetic antioxidant tert-butilhydroquinone was added. After homogenizing the samples, they were placed in a cold chest to make them solid.

After this, portions of the margarine samples were sampled using sterile tubes to perform a stability study 5± 3 °C for the 2 margarine formulations containing olive extract. Furthermore, with the rest of the margarine samples a study of oxidative stability was carried out. The oxidative stability of each of the 8 margarine samples was measured using the Rancimat test at 98°C y 10 l/hour. The oxidative stability is expressed in hours and is an analytical parameter that predicts the time it takes for an olive oil to be rancid. An hour of stability equals approximately one week under appropriate storage conditions. In the case of margarines that equivalence can be somewhat different but the obtained data have a clear comparative value. When a graph with the results was prepared (see Figure 6) a linear correlation between the oxidative stability of the food matrix and the percentage of olive extract added to the food matrix was found. It was found that the margarine Mar:Vit+Ca had increased its stability from 23.8 h (200 mg/Kg TBHQ, 0% olive extract) to 39.4 h, nearly double when 0.13% olive extract was incorporated to the food matrix. On the other hand, the other margarine (Mar: O3 + O6) had increased its oxidative stability from 9.2 h control (200 mg/Kg TBHQ, 0% olive extract) to 21.2 h, i.e. more than double when 0.13% olive extract was incorporated to the food matrix.

The stability of the olive extract incorporated to the 0.02 and 0.13% concentrations in the 2 types of margarines was measured by determining the content of hydroxytyrosol by HPLC between 0 and 6 months stability study at 5±3 °C, finding that the variation in the content of hydroxytyrosol, or losses of the active ingredient in both margarines at the end of 6 months of testing was less than 5% under the test conditions. EXAMPLE 13. Inhibition of enzymes involved in the hydrolysis of carbohydrates in the digestive tract through a pomegranate extract.

A sample of pomegranate extract obtained according to the application EP 1967079 A1 was used to study the in vitro inhibition of the enzymes alpha-amylase and alpha-glucosidase. The enzymes tested were obtained from the following sources: alpha-amylase from pancreas of pig and alpha-glucosidase from Saccharomyces cerevisiae (both purchased from Sigma) and alpha-glucosidase from pig (isolated from pig intestine according to Menakshy et al. 2008).

Measures alpha-amylase activity and alpha-glucosidase were carried out according to procedures supplied by Sigma acquired with enzymes. Briefly, the alpha-glucosidase activity was determined spectrophotometrically at 400 nm, with p-Nitrophenyl α-D-Glucoside (PNP) as substrate and alpha-amylase activity was determined spectrophotometrically at 540 nm, with soluble potato starch as substrate. As a positive control measurements were performed with acarbose as inhibitor.

Measurements were performed in triplicate. In the case of porcine alpha-glucosidase concentration measurements used for positive control acarbose was 125 micrograms / ml, while the pomegranate extract concentration tested was 650 micrograms / ml. In the case of the yeast alpha-glucosidase the tested concentration of pomegranate extract was 1.5 micrograms / ml.

For measures with pig alpha-amylase the positive control measures were with acarbose at 35 micrograms / ml, while the concentration of pomegranate extract tested was 250 micrograms / ml.

A brief summary of the results is shown below:

| Assay | % Pig alfa-glucosidase Activity |
|---|---|
| Control (without inhibitor) | 100.0 ± 3.1 |
| Positive Control (acarbose; 125 micrograms/ml) | 57.0 ± 2.2 |
| Pomegranate extract (650 micrograms/ml) | 88.7 ± 1.4 |

| Assay | % Yeast alfa-glucosidase Activity |
|---|---|
| Control (without inhibitor) | 100.0 ± 3.2 |
| Pomegranate extract (1.5 micrograms/ml. ) | 4.6 ± 2.5 |

| Assay | % Pig alfa-amilase Activity |
|---|---|
| Control (without inhibitor) | 100.0 ± 2.1 |
| Positive Control (acarbose; 35 micrograms/ml) | 41.2 ± 2.3 |
| Pomegranate Extract (250 micrograms/ml.) | 48.0 ± 1.7 |

| | |
|---|---|
| *: Values related to that 100% is the value of enzyme activity obtained without inhibitor. | |

The addition of pomegranate extract at the concentrations tested inhibits alpha-amylase activity and alpha-glucosidase with respect to control, like the addition of acarbose.

A therapeutic approach to decrease postprandial glucose is slow the absorption of glucose by inhibition of enzymes involved in the hydrolysis of carbohydrates in the digestive tract, such as alpha-amylase and alpha-glucosidase. Acarbose, a potent inhibitor of alpha-glucosidase is the active ingredient in drugs used for treatment of type II diabetes mellitus. In view of the results obtained with pomegranate extract at the in vitro inhibition assays one can concluded that pomegranate extract may slow the absorption of glucose by inhibition of enzymes involved in the hydrolysis of carbohydrates in the digestive tract and therefore decrease postprandial glucose and therefore be useful for treatment, co-treatment or prevention of diabetes mellitus type II. Additionally pomegranate extract may be useful for treatment, co-treatment or prevention of obesity through decreased energetic efficiency of food rich in carbohydrates.

EXAMPLE 14. Paraoxonase-1 activation and Inhibition of hydroxymethylglutaryl-CoA reductase by an extract of pomegranate.

A sample of pomegranate extract obtained according to the application EP 1967079 A1 was used to study the inhibition / activation in vitro of the enzyme 3-hydroxy-3-methylglutaryl-CoA reductase (HMGR) and Paraoxonase 1 (PON 1). The enzymes tested were obtained from Sigma and Invitrogen respectively.

Measures HMRG activities and PON 1 were carried out according to procedures supplied by Sigma and Invitrogen with enzymatic kits purchased. As a positive control of HMGR measurements were performed with the pravastatin inhibitor.

Measurements were performed in triplicate. In the case of HMGR concentration measurements used for positive control with pravastatin was 500 nanomolar, while the concentration of pomegranate extract tested was 100 micrograms / ml.

In the case of the PON 1 concentrations tested pomegranate extract were 50 and 100 micrograms / ml.

A summary of the results is shown below:

| Assay | % HMGR activity |
|---|---|
| Control (without inhibitor) | 100.0 ± 3.2 |
| Positive Control (pravastatine) | 9.7 ± 3.4 |
| Product test (pomegranate extract) | 29.0 ± 3.5 |

| | |
|---|---|
| *: Values related to that 100% of value of enzyme activity obtained without inhibitor. | |

| Assay | % PON1 activity |
|---|---|
| Control (without activator) | 100.0 ± 4.3 |
| Pomegranate extract (50 micrograms/ml.) | 107.2 ± 1.2 |
| Pomegranate extract (100 micrograms/ml.) | 128.2 ± 1.9 |

| | |
|---|---|
| *: Values related to that 100% is the value of enzyme activity obtained without activator. | |

The addition of pomegranate extract in the evaluated concentrations inhibits the HMGR activity and stimulates the PON 1 activity.

A therapeutic approach to decrease blood serum cholesterol is to inhibit endogenous cholesterol biosynthesis by inhibiting a key enzyme in the biosynthetic pathway, HMGR. Thus, have been developed from statins, potent inhibitors of HMGR, many drugs to treat hypercholesterolemia. It is interesting to note again that the combination of pomegranate extract with dietary fibre can not only reduce high cholesterol by the bioactivity of the compounds of pomegranate extract, but the dietary fibre (e.g. soluble fibre) once undergo fermentation at the intestine increases the production of short chain fatty acids such as propionate. Propionate is absorbed in the colon through the portal vein and has been shown to inhibit HMGR. On the other hand, curiously statins increase the PON 1 activity in serum, the same protein that pomegranate extract is an activator. It is known that PON 1 protects lipids at the lipoproteins (e.g. in HDL), and at the macrophages, from the oxidative stress that causes their oxidation. PON 1 also presents other antiatherogenic properties, such as reducing the formation of macrophage cell foams, which are involved in the progression of the atherosclerotic lesion. In view of the results obtained with pomegranate extract in the inhibition tests / activation in vitro is concluded that pomegranate extract may act as an inhibitor of HMGR and as activator of PON 1 and therefore be useful for treatment or co-treatment prevention of hypercholesterolemia. Additionally pomegranate extract may be useful for treatment, co-treatment or prevention of the formation of an atherosclerotic plaque in the media and intimae layers of the arteries through mechanisms such as activation of PON 1 among others.

### EXAMPLE 15. Effect of pomegranate extract on the bioavailability of the bioactive components of a broccoli extract in a human intervention study: changes in the urinary excretion of total isothiocyanates including dithiocarbamates.

A commercial sample of broccoli seeds from the Calabrese variety was purchased; 120g sample of seeds was taken and were added over boiling water, approx. 20 volumes of water relative to the weight of the seeds, and were kept boiling around 30 minutes. Then the seeds were homogenised in the cooking water with an Ultra-turrax model T25 at the end of the homogenising treatment, the homogenised mixture was set to boil again, keeping it at 100°C for 10 minutes. Then the coarse solids were removed by filtration of the extract and finally the extract was clarified by centrifugation, saving the supernatant, approximately 460 mL, containing 2.17 mg glucoraphanin/mL. Finally the extract was fractioned into aliquots of 23 mL in sterile Falcon tubes and frozen at -20°C until use. Each fraction of 23 ml contained approximately 50 mg glucoraphanine according to the analysis performed by HPLC determination of this analyte.

The human intervention study was carried out at the same time than the pomegranate extract prebiotic effect intervention study described in Example 4. As already explained, 6 healthy subjects took 1 capsule of pomegranate extract daily at lunchtime, which means 575 mg of extract. The volunteers were told to avoid eating prepared products of prebiotic capacity (such as fructooligosaccharides, inulin, etc.), antibiotics, laxatives and specially products containing probiotics (i.e., unpasteurised yoghourt, fermented milk drinks, etc...). The 6 volunteers began to follow these restrictions on their diets 2 weeks prior to the treatment phase of the intervention study, keeping them on during the 28 days of treatment, where they were to consume one pomegranate extract capsule a day.

At time t=0 (before the start of the administration of pomegranate extract) and t=28 (after the completion of the 28 days treatment with pomegranate extract) the volunteers were administer an aliquot of 23 mL of broccoli seed extract containing approximately 50 mg of glucoraphanin, before lunch.

They were asked to collect the urine from 24h at time 0 (after the 14 days prior to the administration of the pomegranate extract) and after 28 days of administration.

The urine samples were analysed by HPLC after derivatization of the total isothiocyanate according to the cyclocondensation with 1,3-benzodithiole-2-thione method (Kristensen et al., 2007), for the determination of urinary excretion of the total isothiocyanates (ITCs) (which include isothiocyanates in both, free and conjugated form on the isothiocyanates with N-acetylcysteine (called dithiocarbamates, DTCs) of each of the individuals in the 24 h following the ingestion of broccoli extract.

Similarly, an aliquot of broccoli extract of 23 mL containing approximately 50 mg glucoraphanin was thawed and myrosinase was added in order to obtain the corresponding isothiocyanates through the glucosinolates hydrolysis. The sample, after confirming by HPLC that the glucosinolates conversion was complete, was appropriately diluted to determine the total of ITCs according to the cyclocondensation method with 1,3-benzodithiole-2-thione which was used for the determination of the total ITCS from the urine samples. The value obtained was considered to be 100% related to the total excretion of ITCs that could be found in 24 h urine.

The results are summarised below.

**Table 8**

| Patient n° | ITCs*,% at t = 0 | ITCs*,% at t = 28 | Significant Difference^{$} |
|---|---|---|---|
| 1 | 10.1 | 16.4 | p < 0.001 |
| 2 | 22.5 | 15.2 | --- |
| 3 | 30.7 | 32.3 | p < 0.05 |
| 4 | 14.6 | 16.1 | p < 0.05 |
| 5 | 27.1 | 41.6 | p < 0.001 |
| 6 | 14.1 | 50.1 | p < 0.001 |

| | | | |
|---|---|---|---|
| *: Values related to 100% being the obtained value for the total ITCS of the ingestion of 23 mL broccoli extract. $; Statistical analysis performed with triplicate measurements of the real value of total ITCs in urine. | | | |

As shown excretion of total ITCs, including their conjugated forms with N-acetylcysteine, dithiocarbamates, in 24 h urine is significantly higher in 5 of 6 patients (83.3%) after the consumption of pomegranate extract with prebiotic properties capable of stimulating certain populations of microbiota in the colon among which Bifidobacterium and Lactobacillus genera stand out. From the foregoing it can be concluded that the use of a prebiotic, such as pomegranate extract, or a symbiotic can improve the transformation to isothiocyanates of the glucosinolates of a plant extract in the human colon, thus improving the bioavailability of isothiocyanates through the stimulation of colonic microflora.

### EXAMPLE 16. Preparation of a powdered extract from broccoli sprouts..

Several envelopes containing broccoli seeds from the Calabrese variety for use in garden were acquired in a shop. 100 g of the seeds were planted and set to grow in trays in a climatic chamber at 25°C, humidity of 75% and a cycle of 8 h light and 16 h darkness. After 5.5 days the sprouts were harvested, weighing 716 g. Then, the sprouts were added onto boiling water, approximately 10 volumes of water with respect to the weigh of the sprouts, and we kept it boiling for 10 minutes. After that the sprouts were homogenised in the boiling water with a mixer, and the homogenise substance is left for the aqueous extraction to be completed, around 20-40 minutes. Them the coarse solids were removed by filtration of the extract and finally the extract were clarified by centrifugation, the supernatant, around 5500 mL, was reserved. Then it was concentrated by rotaevaporation to obtain 300 mL of concentrated extract containing approximately 3.12 mg glucoraphanin/mL. Finally 60 g of dietary fibre was dissolved in the extract and was dried in a spray dryer. A peristaltic pump was used to feed the spray dryer, it was previously equilibrated with an inlet air temperature of 150°C. The feed rate was adjusted to obtain an outlet air temperature below 90°C. 51.8 g of powder were obtained with a moisture content of 6.72% (thermobalance at 110°C till constant weight) and 1.69% glucoraphanin richness.

### EXAMPLE 17. Functional Food preparation, Rich in edible vegetables as nutritional compounds, through the incorporation of broccoli extract and a dietary fibre and a evaluation on the glucoraphanin stability in the food matrix.

A sample of broccoli extract with a glucoraphanin content of 1.69% (w/w) obtained according to Example 16 was used to prepare the various foods matrices functionalized with the extract.

3 matrices were prepared by adjusting to100 mg glucoraphanin per L of food matrix. To get this, the extract was added specifically to 0 (control), and 0.6 % (functionalize matrices). The dietary fibre used to dry the extract implies that adding 0.6% of extract we are also adding 5.4 g dietary fibre per L of food matrices. 6 samples of product, each of them of approximately 1 kg total weight were prepared.

The matrices used for testing were:
- gazpacho soup
- drink from fruits and vegetables
- courgette puree

The 6 prototypes were pasteurized at 100°C for 15 seconds and aseptically filled in glass containers at a temperature between 85 and 87°C. The 6 glass containers containing the prototypes were dipped into water for rapid cooling. Finally sterile aliquots of 100 ml of each of the products were distributed into glass bottles previously autoclaved and stored at 5±3 °C for evaluating the stability of the broccoli extract in the prepared products. Samples were open after 0. 1, 2 and 3 months, corresponding to the 3 products in order to evaluate the losses of broccoli extract incorporated into food matrices by determining the glucoraphanin content measured by HPLC method.

After 3 months of stability testing, the glucoraphanin losses in the 3 matrices were less than 5% under test conditions (5±3 °C).

### EXAMPLE 18. Differential gene expression in an animal mode

The trial was conducted with three groups of 10 mice; each were fed for 30 days with commercial feed and a daily dose of 12.8 mg of pomegranate extract per mouse (equivalent to a daily intake of 320 mg pomegranate extract / kg body mass) (group 2), or a daily dose of 0.9 mg of olive extract per mouse (equivalent to a daily intake of 22.9 mg olive extract / kg body mass) (group 3) or without pomegranate extract (group 1, control). After the period of administration the mice were sacrificed and each liver was extracted and stored at -80 ° C. In order the results were representative of the group, a pool was prepared with a liver fragment with the same weight of each of the mice in each group. From the same, RNA was isolated, which was subjected to rigorous quality controls so prior to the tests.

The differential gene expression analysis was performed using microarrays. Specifically were used the chips GeneChip ® Mouse Genome 430 2.0 Array (Affymetrix), which permit analysis of the expression of approximately 14,000 genes described in mouse. The chips showed the intensity data dependent on the amount of mRNA for each gene in the form of Log2. From the same, from signal difference between each group versus the control, we calculate the differential gene expression due to treatment with pomegranate extract and to the treatment with olive extract.

The biological interpretation of the results of differential gene expression shown in Examples 18 to 24 was conducted with information obtained from the following databases: RefGene (Reference for genes, proteins and antibodies): http:// refgene.com /; CTD (Toxigenomics The Comparative Database): http://ctd.mdibl.org/, MGI (Mouse Genomics Informatics): http://www.informatics.jax.org/, KEGG (Kyoto Encyclopedia of Genes and Genomes): http://www.genome.jp/kegg/genes.html. The biological function of the genes described, and their involvement in various processes, information derived from both mouse genes, and their counterparts in humans.

### EXAMPLE 19. Properties of pomegranate extract and olive extract, in modulating the immune system, helping to strengthen the body's natural defences.

In the group of mice from Example 18 that took olive extract, an activation on the expression of receptor of interleukin 7 gene (IL7R) of 3.21 times was produced with respect to the control group.

The IL7 is produced by the intestinal epithelial cells and is essential for T lymphocytes proliferation and for the organization of lymphoid tissue associated to the mucosa. The IL7 receptor is found on the surface of intraepithelial lymphocytes of the intestine. The IL7 and its receptor are involved in regulating immune responses in the intestinal mucosa. It has also demonstrated the essential role of IL7R in the formation of the Peyer's patches, which mainly initiates the immune response of the intestine.

Activation in the IL7R gene expression in the intestinal mucosa after the administration of a probiotic in humans has been demonstrated.

In the group of mice from Example 18 that took olive extract, an activation on the expression of ***Dmbt1*** gene (*deleted in malignant brain tumours 1*) of 20.27 times was produced with respect to the control group.

The gene product *Dmbt1* is a glycoprotein containing multiple SRCR domains (*multiple scavenger receptor cysteine-rich*) involved in the recognition of a wide range of pathogenic bacteria. It has been shown *in vitro* a link to enteric *Salmonella* serotype *Typhimurium* and a subsequent bacterial aggregation,which reduces the invasion of epithelial cells. Furthermore, this glycoprotein interacts with at least two different kinds of viruses, HIV and influenza A, resulting in an inhibition of viral infection *in vitro.* The protein also interacts with several molecules involved in immunity such as SP-D, IgA and C1 q. Therefore, the gene product Dmtb1has an important role in innate immunity.

In the group of mice from Example 18 that took olive extract, an activation on the expression of *Tff2* gene (*trefoil factor 2 (spasmolytic protein 1))* of 12.65 times was produced with respect to the control group.

*Tff2* gene product belongs to a family of small proteins (TFF) which are mainly produced at the gastrointestinal tract and are involved in the protection and repair of the mucosa, maintaining the integrity of the gastrointestinal tract.

It has been demonstrated that the administration of a prebiotic mixture in rats and the oral administration of a strain of Lactobacillus in mice promotes the secretion of TFF proteins.

It has been described that the *Dmbt1* gene product could be a receptor of the *Tff2* gene product. Activating the expression of both genes acts synergistically in protecting the gastrointestinal mucosa.

In the group of mice from Example 18 that took olive extract, an activation on the expression of ***Cyp39a1*** gene (*cytochrome P450. family* 39, *subfamily a, polypeptide 1*) of 2.31 times was produced with respect to the control group.

*Cyp39a1* gene product is an enzyme oxysterol 7 α-hidroxilasa involved in cholesterol metabolism. Specifically the enzyme catalyzes the step of 24S-hidroxicholesterol into 7α,24-dihidroxicholesterol into the pathway of conversion of cholesterol into bile acids in the liver. Bile acid provides the intestinal mucosa protection against bacteria. In the distal intestine the antibacterial ability of bile acids is due to activation of the FXR receptor (*Farnesoid X Receptor*) which induces the expression of genes whose products prevent bacterial growth, and promote the gastrointestinal epithelium integrity.

In the group of mice from Example 18 that took pomegranate extract an activation on the expression of Scara5 gene (scavenger receptor class A, member 5 (putative) of 2.46 times was produced with respect to the control group.

In the group of mice from Example 18 that took olive extract an activation on the expression of Scara5 gene (scavenger receptor class A, member 5 (putative) of 3.37 times was produced with respect to the control group.

Scara5 gene product is a protein belonging to the family of scavenger receptor type, which is involved in innate immunity related with pathogen recognition because it has demonstrated its ability to bind to a variety of these. Scara5 gene is expressed in epithelial cells and also has binding pathogens capacity.

In the group of mice from Example 18 that took olive extract an activation on the expression of ***Cfd*** gene (*complement factor D (adipsin)*) of 2.57 times was produced with respect to the control group.

*C̅f̅d̅* gene product is a serine protease (factor D of the complement) which is involved in the activation of the complement system, specifically in the alternative pathway, involved in the recognition and elimination of pathogens. The complement factor D is involved in the formation of the C3bBb complex (C3 convertase of the alternative pathway). The C3 convertase is involved in the opsonization of pathoges and neutralization of viruses.

In the group of mice from Example 18 that took olive extract an activation on the expression of ***C7*** gene (*complement component 7*) of 1.85 times was produced with respect to the control group.

C7 gene product is a glycoprotein (complement factor C7) involved in the activation of the classical pathway of the complement system. In humans, the deficiency of the C7 protein causes abnormalities in processes such as neutrophil chemotaxis, phagocytosis and opsonization.

### EXAMPLE 20. Properties of the pomegranate extract and of the olive extract in the regulation of lipid metabolism.

In the group of mice from Example 18 that took olive extract, an activation on the expression of ***Cyp39a1*** gene (*cytochrome P450. family 39*, *subfamily a, polypeptide 1*) of 2.31 times was produced with respect to the control group.
*Cyp39a1* gene product is an enzyme oxysterol 7 α-hidroxilasa involved in cholesterol metabolism. Specifically the enzyme catalyzes the step of 24S-hidroxicholesterol into 7α,24-dihidroxicholesterol into the pathway of conversion of cholesterol into bile acids in the liver.
The mRNA of this enzyme is expressed constitutively in the liver. Bile acid biosynthesis is the more important pathway to remove excess cholesterol in mammals

In the group of mice from Example 18, who took an extract of pomegranate a repression on the expression of Serpine1 gene (serpin peptidase inhibitor, clade E, member 1) of 3.51 times was produced with respect to the control group.

In the group of mice from Example 18 that took olive extract, an activation on the repression on the expression of Serpine1 gene (serpin peptidase inhibitor, clade E, member 1) of 3.49 times was produced with respect to the control group.

The product of *Serpine1* gene is called inhibitor of plasminogen activator type 1 (PAI-1). It has been shown that a diet rich in cholesterol in mice produced an activation of serpine1 expression in liver. The PAI-1 plasmatic levels in human with hypercholesterolemia are high and it has been shown that statins inhibit serpine1 gene expression in human endothelial cells. Statins are drugs that inhibit the enzyme HMG CoA reductase indicated to reduce cholesterol levels in patients with hypercholesterolemia. An inhibitor of intestinal absorption of dietary cholesterol (ezetimibe), also indicated in hypercholesterolemia, represses serpine1 gene expression in aorta and adipose tissue of mice.

In the group of mice from Example 18 that took olive extract, an activation on the expression of ***Cyp17a1*** gene (*cytochrome P450. family 17*, *subfamily a*, *polypeptide 1*) of 4.27 times was produced with respect to the control group
*Cyp17a1* gene product is an enzyme involved in steroid hormone biosynthesis from cholesterol. Specifically the enzyme catalyzes the 17α-hydroxylation of C21 steroids, pregnolona and progesterone, and the subsequent processing (17.20 lyase) for C19 steroids dehydroepiandrosterone and androstenedione obtention, respectively. Low levels of CYP17A1 have been linked to hypertension.

Therefore it is possible co-treatment of hypercholesterolemia by administering an active dose of an extract of pomegranate and a statin in doses lower than prescribed when treatment is carried out only with the statin.

### EJEMPLO 21. Regulation of mineral absortion of the pomegranate extract and of the olive extract.

In the group of mice from Example 18 that took olive extract, an activation on the expression of ***Mt4*** gene (*metallothionein 4*) of 9.1 times was produced with respect to the control group.

*Mt4* gene product is a cytoplasmic protein which binds to minerals such as copper and zinc. Metallothioneins involved in homeostasis of Zn and Cu and in the detoxification of them when they reach high concentrations.

### EXAMPLE 22. Properties of the pomegranate extract and of the olive extract in the treatment, co-treatment and prevention of different oncological processes / diseases.

In the group of mice of Example 18, who took an extract of pomegranate a repression of the expression of oncogenes jun, myc and fos of 3.04, 3.27 and 6.43 times respectively was produced with respect to the control group.

In the group of mice from Example 18 that took olive extract, repression of the expression of oncogenes jun, myc and fos of of 2.22, 3.75 and 4.31 times respectively was produced with respect to the control group. The *jun* and *fos* products are transcription factors that dimerize to form the transcription complex called AP-1 (Activating Protein-1). AP-1 is a transcription factor that regulates expression of genes induced by growth factors and tumour promoters. Overexpression of oncogenes *Jun* and / or *fos* is associated with several cancers such as breast, ovarian, colon, osteosarcoma, cervical, lung and bladder cancer. Therefore, AP-1 was used as a target for chemotherapeutic treatment of cancer.

*Myc* oncogene product is a protein that regulates the expression of the transcription factor E2F and phosphatase responsible for activation of Cdc cyclins, which are involved in cell cycle regulation. The *myc* oncogene is overexpressed in many human cancers, including pancreatic , cervical, breast and colon cancer. *Myc* oncogene product is also used as target for cancer treatment.

A study in humans showed that in the intestinal mucosa there are a repression in the expression of oncogenes *jun, fos* and *myc* after infusion of the membrane with a strain of *Lactobacillus.*

In the group of mice of Example 18, who took extract of pomegranate a repression on the expression of the gene ***Adamts1*** (a disintegrin-like and metalloproteinase (reprolysin type) with thrombospondin type 1 motif) of 2.04 times was produced with respect to the control group.

In the group of mice of Example 18, who took extract of olive a repression on the expression of the gene ***Adamts1*** (a disintegrin-like and metalloproteinase (reprolysin type) with thrombospondin type 1 motif) of 1.84 times was produced with respect to the control group.

*Adamts1* gene product is a protein that has a metalloproteinase domain and disintegrin domain. This protein is involved in inflammatory processes and the development of cancer cachexia as tested in animal models of colon cancer. It has been demonstrated overexpression of *Adamts1* gene in breast cancer with high metastatic activity. It has been speculated that overexpression of this gene might promote tumour growth by recruiting fibroblasts.

In the group of mice of Example 18, who took an extract of pomegranate a repression on the expression of the gene *ATF3 (activating transcription factor 3)* of 4.34 times was produced with respect to the control group.

In the group of mice from Example 18 that took olive extract, a repression on the expression of receptor of **ATF3** gene *(activating transcription factor 3)* of 4.27 times was produced with respect to the control group.

*ATF3* gene product is a transcription factor expressed in conditions of stress and DNA damage in various tissues. In many breast tumours has been reported overexpression of ATF3. This protein is used as a marker of prostate cancer since its involvement has been shown in developing this type of cancer and is therefore a potential therapeutic target.

In the group of mice from Example 18 that took olive extract, a repression on the expression of receptor of ***Dmbt1*** gene (*deleted in malignant brain tumors 1*) of 20.27 times was produced with respect to the control group.

The gene product Dmbt1 is a membrane glycoprotein with function of receptor having domains of type SRCR (cysteine-rich domains scavenger type) whose lack of function was associated with breast cancer. Furthermore, it is considered a tumor suppressor gene of colon, digestive and lung cancer.

In the group of mice from Example 18 that took olive extract, an activation on the expression of ***Tff2*** gene (*trefoil factor 2 (spasmolytic protein 1*)) of 12.65 times was produced with respect to the control group.

Tff2 gene product belongs to a family of small proteins (TFF) which are mainly produced at the gastrointestinal tract and are involved in the protection and repair of the mucosa, maintaining the integrity of the gastrointestinal tract.

Repression in the expression of TFF2 gene is associated with the proliferation of gastrointestinal cancer.

In the group of mice from Example 18 that took olive extract, an activation on the expression of ***Nupr1*** gene (*nuclear protein, transcriptional regulator, 1*) of 7.9 times was produced with respect to the control group.

Nupr1 gene product is a nuclear protein (NURP1 or p8) involved in various biological processes by regulating transcription. It has been shown in vitro that a overexpression of this protein reduces the growth of prostate cancer cells. Therefore, the protein p8 has been described as a prostate tumor suppressor. It has been demonstrateda high Nurp1 gene overexpression mediated by the action of 1,25-dihydroxyvitamin D3, a potent inhibitor of breast cancer cells in vivo and in vitro, representing a involvement of the p8 protein in the inhibition mechanism.

In the group of mice from Example 18 that took olive extract, an activation on the expression of ***FAM107A*** gene (*family with sequence similarity 107, member A*) of 7.17 times was produced with respect to the control group.

FAM107A gene product is a nuclear protein called TU3A involved in cell cycle regulation. Was identified as a tumor suppressor protein in kidney cancer. Has been demonstrated a repression in FAM107A gene expression in various types of cancer, including prostate cancer.

In the group of mice from Example 18 that took a pomegranate extract an activation on the expression of ***Ddit4*** gene (*DNA-damage-inducible transcript 4*) of 2.92 times was produced with respect to the control group.

In the group of mice from Example 18 that took an olive extract an activation on the expression of ***Ddit4*** gene (*DNA-damage-inducible transcript 4)* of 6.08 times was produced with respect to the control group.

*Ddit4* gene product is a protein called RTP801 or REDD1, which inhibits the pathway mTOR/S6K1, involved in cell proliferation. Inhibitors of this route are being evaluated as cancer therapy. In mice has been described that the gene *Ddti4* deficiency promotes tumour growth, while in humans has been described repression of the gene in various cancers.

In the group of mice of Example 18, who took an extract of pomegranate a repression on the expression of ***Egr1*** gene *(early growth response 1)* of 2.61 times was produced with respect to the control group.

In the group of mice of Example 18, who took an olive extract a repression on the expression of ***Egr1*** gene of 1.72 times was produced with respect to the control group.

The *Egr1* gene product is a transcription factor involved in various cellular processes and its involvement has been demonstrated in cell growth and survival of prostate cancer. In animal models of prostate cancer has shown that lack of Egr1 gene retards tumour growth.

In the group of mice of Example 18, who took an extract of pomegranate a repression on the expression of ***Sox9*** *gene (SRY (sex Determining Region Y*)-*box 9*) of 2.45 times was produced with respect to the control group.

In the group of mice of Example 18, who took an olive extract a repression on the expression of ***Sox9*** *gene* of 3.62 times was produced with respect to the control group.

The *Sox9* gene produces a protein that acts as a transcription factor with DNA-binding domain type HMG *(High Mobility Group).* It has been shown an implication of Sox9 gene product in the proliferation of pancreatic cancer and overexpression of the same in different cell lines of colon cancer.

In the group of mice of Example 18, who took an extract of pomegranate a repression on the expression of interleukin 1 alpha gene **(*IL1α*)** of 2.97 times was produced with respect to the control group.

In the group of mice of Example 18, who took an olive extract a repression on the expression of interleukin 1 alpha gene of 2.14 times was produced with respect to the control group.

The IL1α is a cytokine involved in inflammatory processes. The *IL1*α gene is overexpressed in various cancers, including lung cancer, colon and melanoma. In colon cancer IL1α stimulates the cell migration and angiogenesis and its expression is induced by prostaglandin E2. In a study in humans showed that in the intestinal mucosa occurs a repression in the IL1α gene expression after the infusion of the membrane with a strain of *Lactobacillus.*

In the group of mice from Example 18, who took an extract of pomegranate a activation on the expression of ***Gadd45b*** gene *(growth arrest and DNA-damage-inducible 45 beta)* and ***Gadd45g*** *gene (growth arrest and DNA-damage-inducible 45 gamma)* of 2.24 and 2.57 times respectively, were produced with respect to the control group.

In the group of mice from Example 18, who took an olive extract a activation on the expression of ***Gadd45b*** gene *(growth arrest and DNA-damage-inducible 45 beta)* and ***Gadd45g*** *gene* of 2.43 and 1.73 times respectively, were produced with respect to the control group.

The products of *Gadd45g and Gadd45b* genes are proteins related with cell cycle control. In mice models of melanoma have been shown that a lack of function of *Gadd45b* produces higher tumour growth. The product of this gene is required for activation of p38 kinase. The p38 protein is involved in tumour suppression. The expression of *Gadd45g and Gadd45b genes* is repressed in various cancers.

### EXAMPLE 23. Properties of the pomegranate extract and of olive extract in the treatment, co-treatment and prevention of colon cancer.

In the group of mice of Example 18, who took an extract of pomegranate a repression of the expression of oncogenes jun, myc and fos of 3.04, 3.27 and 6.43 times respectively was produced with respect to the control group.

In the group of mice from Example 18 that took olive extract, repression of the expression of oncogenes jun, myc and fos of of 2.22, 3.75 and 4.31 times respectively was produced with respect to the control group.

The *jun* and *fos* products are transcription factors that dimerize to form the transcription complex called AP-1 (Activating Protein-1). AP-1 is a transcription factor that regulates expression of genes induced by growth factors and tumour promoters.

Overexpression of oncogenes *jun* and / or fos is associated with colon cancer.

M*yc* oncogene product is a protein that regulates the expression of the transcription factor E2F and phosphatase responsible for activation of Cdc cyclins, which are involved in cell cycle regulation. The *myc* oncogene is overexpressed in colon cancer. Both AP-1 and *myc* oncogene product are also used as target for cancer treatment.

A study in humans showed that in the intestinal mucosa there are a repression in the expression of oncogenes *jun,* fos and *myc* after infusion of the membrane with a strain of *Lactobacillus.*

In the group of mice of Example 18, who took an extract of pomegranate a repression on the expression of ***Fam84A*** *gene (family with sequence similarity 84, member A*) of 2.53 times was produced with respect to the control group.

In the group of mice of Example 18, who took an olive extract a repression on the expression of *Fam84A gene* of 2.52 times was produced with respect to the control group.

The product of Fam84A gene is a protein known as NSE1 *(neurologic sensory protein 1)* that is located in the sub cellular membrane and is involved in cell motility. The expression of this gene is activated in colon cancer. NSE1 protein might be involved in the mobility of colon cancer cells and thus participate in the progression of this cancer.

In the group of mice of Example 18, who took extract of pomegranate a repression on the expression of the gene ***Adamts1*** (a disintegrin-like and metalloproteinase (reprolysin type) with thrombospondin type 1 motif) of 2.04 times was produced with respect to the control group.

In the group of mice of Example 18, who took extract of olive a repression on the expression of the gene ***Adamts1*** of 1.84 times was produced with respect to the control group.

*Adamts1* gene product is a protein that has a metalloproteinase domain and disintegrin domain. This protein is involved in inflammatory processes and the development of cancer cachexia as tested in animal models of colon cancer.

In the group of mice from Example 18 that took olive extract, a repression on the expression of receptor of ***Dmbt1*** gene *(deleted in malignant brain tumors 1*) of 20.27 times was produced with respect to the control group.

The gene product Dmbt1 is a membrane glycoprotein with function of receptor having domains of type SRCR (cysteine-rich domains scavenger type). This protein is considered a tumor suppressor gene of colon

In the group of mice of Example 18, who took an extract of pomegranate a repression on the expression of ***Sox9*** *gene (SRY (sex Determining Region Y*)-*box 9)* of 2.45 times was produced with respect to the control group.

In the group of mice of Example 18, who took an extract of olive a repression on the expression of ***Sox9*** *gene* of 3.62 times was produced with respect to the control group.

The *Sox9* gene produces a protein that acts as a transcription factor with DNA-binding domain type HMG *(High Mobility Group).* It has been demonstrated overexpression of Sox9 gene in various cell lines of colon cancer.

In the group of mice of Example 18, who took an extract of pomegranate a repression on the expression of interleukin 1 alpha gene *(***IL1**α*)* of 2.97 times was produced with respect to the control group.

In the group of mice of Example 18, who took an extract of olive a repression on the expression of interleukin 1 alpha gene of 2.14 times was produced with respect to the control group.

The IL1α is a cytokine involved in inflammatory processes. The IL1α gene is overexpressed in colon cancer. In colon cancer IL1α stimulates the cell migration and angiogenesis and its expression is induced by prostaglandin E2. In a study in humans showed that in the intestinal mucosa occurs a repression in the IL1α gene expression after the infusion of the membrane with a strain of *Lactobacillus.*

### EXAMPLE 24. Properties of the pomegranate extract and of the olive extract in the treatment of obesity and diabetes mellitus.

In the group of mice from Example 18, who took an extract of pomegranate a repression on the expression of Serpine1 gene (serpin peptidase inhibitor, clade E, member 1) of 3.51 times was produced with respect to the control group.

In the group of mice from Example 18, who took an extract of olive a repression on the expression of Serpine1 gene of 3.49 times was produced with respect to the control group.

The product of *Serpine1* gene is called inhibitor of plasminogen activator type 1 (PAI-1). In diabetes type 1 and type 2 have been observed high plasma levels of PAI-1 protein, most pronounced in the case of type-2 diabetes. Such high levels in this type of diabetes are due to the direct effect of glucose in the synthesis of PAI-1 in the arteries and the effect of insulin in the synthesis of PAI-1 in liver. The protein PAI-1 inhibits insulin signalling by binding to and stabilization with vitronectin. Thiazolidinediones or glitazones are agonists drugs of the receptor PPAR-γ indicated in type-2 diabetes, which cause a decrease in the levels of PAI-1. Has been described a correlation between high glucose levels, even within the normal range, in healthy people and levels of PAI-1. An activation in the expression of serpine1gene in liver of diabetic rats has been observed.

Elevated levels of PAI-1 in plasma are a major cause of inactivation of the fibrinolytic activity, which has for many years associated with obesity. In cases of weight loss in obese patients has been reported a decrease in plasma levels of PAI-1, which are recovered in the case of weight gain.

In the group of mice from Example 18, who took an extract of olive an activation on the expression of ***ppargc1a*** gene (*peroxisome proliferative activated receptor, gamma, coactivator 1*) of 3.14 times was produced with respect to the control group.

*Ppargc1a* gene product is a transcription factor that participates in the regulation of genes involved in energy metabolism by activating PPAR-y receptor. This hormone receptor has an important role in the regulation of metabolism of carbohydrates and lipids, by increasing insulin sensitivity.

Substances agonists of receptor PPAR-y are being used as drugs for the treatment of type 2 diabetes. Among these are the thiazolidinediones. As pharmacological activators of receptor PPAR-γ, thiazolidinediones exert an important metabolic effect improving glucose metabolism (lower production and increased clearance of glucose) and decreasing insulin resistance associated with obesity, metabolic syndrome and type 2 diabetes mellitus.

In addition, these drugs have significant effects on plasma lipids, being useful in the treatment of dyslipidemias. It has been shown that hydroxytyrosol produces a *ppargc1a* gene activation in adipocytes. The expression of this receptor gene is repressed in mice with type 1 diabetes.

In the group of mice from Example 18, who took an extract of olive an activation on the expression of ***adipoq*** gene (*adiponectin*) of 2 times was produced with respect to the control group.

The product of gene *adipoq* is a cytokine (adiponectin) synthesized mainly in adipose tissue and abundant in plasma. In animal models and humans have demonstrated a low level of adiponectin in plasma in diabetes. Plasma levels of these cytokines are increased with the treatment of diabetes with thiazolidinediones.

Adiponectin is also related to obesity. In obese mice a repression of *adipoq* gene has been described. In humans, plasma levels of adiponectin are lower in obese patients. The levels of this cytokine in women are inversely related to BMI. It has been speculated that adiponectin, or substances that stimulate their production, could be used to treat type 2 diabetes and obesity.

In the group of mice from Example 18 that took a pomegranate extract an activation on the expression of ***Lpin1*** gene (Lipin 1) of 2.35 times was produced with respect to the control group.

In the group of mice from Example 18 that took olive extract a represion on the expression of ***Lpin1*** gene of 2.66 times was produced with respect to the control group.

*Lpin1* gene product is a protein belonging to the family of the lipines that was described in mice whose lack of function produced lipodystrophy. This protein participates in adipogenesis and metabolism of triglycerides, also participating with PPARGC1A gene product in transcriptional activation in liver of PPAR-γ receptor. *Lpin1* gene levels in adipose tissue are inversely related to insulin resistance. This tissue has shown an increase in *Lpin1* gene expression following treatment with thiazolidinediones, drugs whose mechanism of action is an increase in insulin sensibility.

In the group of mice from Example 18 that took an olive extract an activation on the expression of ***lepr*** gene (leptin receptor) of 4.25 times was produced with respect to the control group.

Lepr gene product is the receptor for leptin, a hormone involved in regulating metabolism. Leptin is involved in regulation of adipose tissue, acting at the level of central nervous system, where it appears to decrease food intake and increase energy expenditure. In tests conducted in mice that did not produce leptin or its receptor was shown that obesity is developed. In the case of obese mice that did not produce leptin, they were successfully treated with this hormone. After treatment there was an increase of gene expression of leptin receptor in the liver. Therefore, the liver appears to be an important role in regulating the circulating levels of leptin.

### EXAMPLE 25. Differential gene expression using quantitative RT-PCR

The results shown in Examples 19 to 24 of differential gene expression associated with the results of the microarray (see Example 18) were confirmed by calculating differential gene expression by quantitative RT-PCR. From the same RNA isolated from the liver of mice was performed RT-PCR with SYBR Green. Relative quantification was carried out using as a reference gene GAPDH mouse, with constitutive expression. The method used for relative quantification was described by Pfaffl, 2001.

### EXAMPLE 26. Characterization of the ellagitannins of pomegranate extracts by HPLC-DAD and mass spectrometry detectors.

In order to further characterize the ellagitannins present in **extracts of pomegranate** we proceeded to **subfractionation of the extracts,** for a subsequent analysis of each of the fractions of interest to detect the maximum possible compounds.

In order to get this, a standardized method comprising:
- **A first fractionation by ASE,** which applies a sequence of solvents from low to high degree of polarity:
   - Step defatting with Hexane (3 washes) _ ASE Hexane Fraction
   - Step extraction with ethyl acetate (3 washes) _ Ethyl Acetate Fraction ASE
   - Step extraction with acetone (3 washes) _ ASE Acetone Fraction
   - Step extraction with acetone: water (3 washes) _ Fraction Acetone: Water ASE
- And a **second step** which performs a **manual extraction liquid-liquid (LLE)** with ethyl acetate (3 washes) applied to the fraction acetone: water obtained in the ASE, after removal of acetone:
   - Ethyl Acetate Fraction LLE
   - Water Fraction LLE

Subsequently we have proceeded to **characterize analytically the various isolated fractions**, using liquid chromatography (HPLC) for analysis of polar fractions. At the HPLC methodology has developed a chromatographic method of screening in order to identify the maximum number of compounds from different families to be found in the sample. For identification of compounds has been used diode array detection (DAD) as well as mass spectrometry detectors: time of flight (TOF) and ion trap (IT).

A total of 18 compounds were determined by the procedure described above and shown below in Table 9.

**Tabla 3. no ha podido confirmar eu a partir de la fragmentation mediarde ITMS; AcE11. acetanode elle obenda madame ASE Acet ta aceto mediante ASE; AcEt2: fraccon acetan de elle de la L.E realizada a la traccier acet mediante ASE; H₂O: traccion agua de la LLE realizada a la traccion aceagua otrandaASE.**

| N° | Compost | TOF-MS | | IT-MS | | | AcEt 1 | AcEt | Acetona:Agua | |
|---|---|---|---|---|---|---|---|---|---|---|
| | | tr | m/z | tr | Precursor | m/z | | | AcEt2 | H₂O |
| 1 | 2,3-O-(S)-hexahydroxydiphenoyl-D-glucose | 0.4 | [M-H] 481.0523 | 1.6 | 481 | 420.7, 3007 | x | x | | x |
| 2 | punicalin | 0.6 | [M-H]° 781.0426 | 29 | 781 | 720.0 600.9 | | x | | x |
| 2 | *Eilagic acid derivative* | 0.6 | 707.0567 | 3.1 | | 783.0, 698.5, 633.0, 613.1 300.6 | | x | | x |
| 2 | pedunculagin | 0.6 | [M-H]⁻ 783.0373 | 3.1 | 783. | 720.9, 600.9, 480.9. 300.7 | x | x | x | |
| 2 | *Ellagic acid derivative* | | | 4.1 | 333 | 916.0, 781.0, 721.0, 601.0 | | x | x | |
| 3 | 0-punicalagin | 0.9 | | 4.8 | | 721.0. 800.9 | x | x | x | x |
| 4 | | | [M-H] 0083.0131 | 5.7 | 1083 | 781.0, 300.7, 274.8 | x | x | x | x |
| 5 | *Ellagic acid derivative* | 1.5 | 951.0337 | 7.0 | 951 | 907.3, 783,7. 605 | x | x | x | x |
| 6 | β-punicalagin | 1.8 | [M-H] 1083.0129 | 8.7 | 1083 | 761.0. 721.0. 6008 | x | x | x | x |
| 6 | *Ellagic acid derivative* | | | 8.9 | 851 | 907.3. 783.1. 605.0 | | x | | |
| 7 | *Ellagic acide derivative* | 2.6 | 467.0221 | 11.7 | 467 | 457.8, 390.8.300.7, 274.7 | x | x | x | |
| 7 | *Ellagic acid derivative* | 2.6 | 635.0396 | 11.7 | 935 | 97.0, 6330 571.0,300.7 | x | x | x | |
| 8 | catechin | 2.6 | [M-H] 288.0645 | 12.0 | 289 | 244.7,204.7 | x | x | x | |
| 8 | *Ellagic acid derivative* | 2.8 | 799.0307 | 12.4 | 799 | 781.0, 478.9. 300.7 | x | x | x | |
| 9 | 2-o-galloylpunicalin | 3.1 | [M-H] 933.0237 | 13.2 | 933 | 915.0. 620.9, 2508, 300.8 | | | x | |
| 9 | 6-O-galloy}-2,2-hexahydroxydiohenoyl-{σ/β)-O gluccopyranose (7: | 3.1 | [M-H] 633.0493 | 13.3 | | | x | x | x | |
| 9 | *Ellagic acid derivative* | 3.1 | 783.070 | 13.4 | | 765.1, 300.5, 274.8 | x | x | | |
| 10 | 1,2-d-O-galloyl-4.6-O-(S)-hexahdroxydiphenoyl-β-D-glucopyranoside | 3.5 | [M-H]⁻ 785.0531 | 14.9 | | | x | x | x | |
| 11 | 6-O-galloyl-2,3-hexahydroxydiphenoyl-{σ/β)-D glucopyranose | 5.3 | [M-H] 633.10500 | 19.3 | 633 | 462.8, 418.6, 300.7, 274.8 633->463: 3007 | x | x | x | |
| 12 | unknown | 5.6 | 469.0059 | 20.7 | | 424.8, 298.7 | | | x | |
| 13 | *Ellagic acid derivative* | 6.1 | 951.0338 | 21.7 | 951 | 933,2, 65.1 613.0, 444.9. 300.8 951->933: 915.9, 765.0, 612.9. 444.8. 300.7 | x | x | x | |
| 14 | Ellagic acid glucoside | 6.4 | [M-H]⁻ 463.0516 | 22.7 | 463 | 300.7 | x | x | x | x |
| 15 | 1,2-dj-O-galloyl-4,6-O-[S]-hexahydroxydiphenoyl-β-D-glucopyranoside | 6.9 | [M-H]⁻ 785.0531 | 22.4 | 785 | 764. 614 300.7, 274.8, 246.9 | | | x | |
| 16 | *Ellagic acid derivative* | 8.9 | 985.0502 | 23.9 | 966 | 933.5, 613.1, 445.0, 300.9 | | | x | |
| 17 | *Ellagic acid rhamnoside | 7.5 | [M-H]⁻ 447.0572 | 25.9 | 447 | 299.7 | x | x | x | x |
| 18 | Ellagic acid | 7.6 | *[M-H]⁻ 300.9915 | 26.2 | 301 | 256.7, 228.7. 194.6 | x | x | x | |

### BIBLIOGRAPHY

Collins MD, Gibson GR. Probiotics, prebiotics and synbiotics: approaches for the nutritional modulation of microbial ecology. 1999. American Journal of Clinical Nutrition 69: 1052s-1057s.
Gibson GR, Probert HM, Van Loo J, Rastall RA, Roberfroid MB Dietary modulation of the colonic microbiota: updating the concept of prebiotics. 2004. Nutrition Research Reviews 17: 259-275.
Kristensen M, Krogholma KS, Frederiksen H, Duus F, Cornett C, Bugel SH, Rasmussen SE. Improved synthesis methods of standards used for quantitative determination of total isothiocyanates from broccoli in human urine. 2007. Journal of Chromatography B, 852: 229-234.
Liong MT. Roles of Probiotics and Prebiotics in Colon Cancer Prevention: Postulated Mechanisms and In-vivo Evidence.2008. International Journal of Molecular Sciences 9(5):854-63.
Liu W, and Saint D.A. A new quantitative method of real time reverse transcription polymerase chain reaction assay based on simulation of polymerase chain reaction kinetics. 2002. Anal Biochem. 302(1): 52-9.
Menakshi B, Smita SZ, Shobha YB, Ameeta RK and Bimba NJ. 2008. Antidiabetic Indian Plants: a Good Source of Potent Amylase Inhibitors.
Pfaffl MW. A new mathematical model for relative quantification in real-time RT-PCR. 2001. Nucleic Acid Research 29(9):e45.
Pool-Zobel BL. Inulin-type fructans and reduction in colon cancer risk: review of experimental and human data. 2005. British Journal of Nutrition 93 Suppl 1:S73-90.
Venter CS. Prebiotics: an update. 2007. Journal of Family Ecology and Consumer Sciences 35: 17-25.

## Claims

1. Pomegranate plant extract having a polyphenol content, with antimicrobial properties, of at least 5% (w / w) (measured as total phenols and expressed as gallic acid equivalent), a punicalagins content of at least 2% (w / w), and having a punicalagins / free ellagic acid (% w / w) ratio, in the range of 10/1 to 35/1, for use as a prebiotic.

2. Pomegranate plant extract for use according to claim 1, wherein the solubility of said plant extract in water is at least 3% (w / w).

3. Pomegranate plant extract for use according to claim 1, **characterized in that** said pomegranate extract has a punicalagins content of at least 2% (w / w), a citric acid content of at least 0.5% (w / w) and / or an ascorbic acid content of at least 0.05% (w /w).

4. Pomegranate plant extract, for use according to claim 1, **characterized in that** said pomegranate extract has a punicalins content of at least 1.5% (w / w), and has a ratio punicalins / free ellagic acid (% w / w) in the range of 10/1 to 35/1.

5. Composition comprising at least one pomegranate plant extract according to any of claims 1 to 4 for use as a prebiotic.

6. A food, including a composition according to claim 5.

7. A food, according to claim 6, **characterized in that** said food is a refreshing drink of fruits, with a content of at least 10% apple juice, combined or not with other fruit juices, with a ascorbic acid content from 0 to 9000 ppm, with a citric acid content between 0% and 5% and with a pomegranate extract content between 50 ppm and 10000 ppm.

8. A food, according to claim 6, **characterized in that** said food is a refreshing drink of fruits, with a content of at least 12 % grape juice, combined or not with other fruit juices, with a ascorbic acid content from 0 to 9000 ppm, with a citric acid content between 0% and 5% and with a pomegranate extract content between 50 ppm and 10000 ppm.

9. A food according to any of the claims 6 to 8, containing a probiotic bacterial strain.

10. A food according to claim 9, for use as a symbiotic.

11. A symbiotic food according to claim 9, **characterized in that** said food contains an amount of probiotic bacteria in a range between 102 a 1010 CFU / g of composition.

12. A symbiotic food according to claim 11, **characterized in that** said probiotic bacteria belong to the genera Bifidobacterium and / or Lactobacillus.

13. A symbiotic food according to claim 12, wherein the probiotic strain contained in said composition is Lactobacillus casei DN 114-001, and/or Bifidobacterium animalis DN-173 010.

14. A food according to any of the claims 6 to 13 for use to enhance the production of urolithins A and B in the human colon lumen.

15. A food according to any of the claims 6 to 14, for use in a treatment, co-treatment or prevention of cancer diseases such as prostate cancer, colon cancer and pancreatic cancer.

16. A food according to any of the claims 6 to 9, for use in treatment to stimulate the growth of bacteria of the genus Bifidobacterium and Lactobacillus in the human colon, helping to strengthen the body's natural defences.

17. A food according to any of the claims 6 to 9, for use in a treatment, co-treatment or prevention of metabolic syndrome.

18. A food according to any of the claims 6 to 9, for use in treatment, co-treatment or prevention of one or more disorders selected from type II diabetes mellitus and obesity.

19. A food according to any of the claims 6 to 9 for use in a treatment, co-treatment or prevention of hypercholesterolemia.

20. A food according to any of the claims 6 to 9 for use in a treatment, co-treatment or prevention of the formation of an atherosclerotic plaque in the middle and intimate layers of the arteries.

21. A food according to any of the claims 6 to 9 for use in a treatment, co-treatment or prevention of cardiovascular diseases.

22. A food according to any of the claims 6 to 9 for use in treatment, co-treatment or prevention of colon cancer.

23. A food according to any of the claims 6 to 9 further comprising a plant extract containing glucosinolates.

24. A food according to claim 23 for use to stimulate human colonic microflora.

25. A food according to claim 23, **characterized in that** said said plant extract containing glucosinolate is a broccoli extract.

26. A food according to claim 25 wherein the broccoli extract is prepared from the seeds of broccoli.

27. A food according to claim 25 wherein the broccoli extract is prepared from the broccoli sprouts.

28. A food according to claim 23, wherein the majoritarian glucosinolate is the glucoraphanin.

29. A food according to any of claims 23 to 28, comprising as main nutritional components horticultural products, vegetable and / or fruits, which is selected from the gazpacho soup, vegetable soups, vegetable purees, vegetable juices with or without fruit juice, canned vegetables, technologically modified derivatives of the above mentioned foods or mixtures of two or more of the same.

30. A food according to any of the claims 23 to 29 for use in a treatment, co-treatment or prevention of cardiovascular diseases.

31. A food according to any of the claims 23 to 29 for use in a treatment, co-treatment or prevention of arterial hypertension.

32. A food according to any of the claims 23 to 29 for use in a treatment, co-treatment or prevention of the formation of an atherosclerotic plaque in the middle and intimate layers of the arteries.

33. A food according to any of the claims 23 to 29, for use in a treatment, co-treatment or prevention of metabolic syndrome.

34. A food according to any of the claims 23 to 29, for use in a treatment, co-treatment or prevention of cancer diseases such as prostate cancer, colon cancer, breast cancer, bladder cancer, kidney cancer, pancreatic cancer, lung cancer and skin cancer.

35. A food according to any of the claims 6 to 9 further comprising an olive plant extract, with a hydroxytyrosol content of at least 0.5% (w / w) and a purity of at least 40% (by HPLC 280 nm).

36. A food according to any of the claims 23 to 25 further comprising an olive plant extract, with a hydroxytyrosol content of at least 0.5% (w / w) and a purity of at least 40% (by HPLC 280 nm).

37. A food according to any of the claims 35 to 36, for use in a treatment, co-treatment or prevention of cardiovascular diseases, arterial hypertension, the formation of an atherosclerotic plaque in the middle and intimate layers of the arteries, metabolic syndrome, oncological diseases such as prostate cancer, colon cancer, breast cancer, bladder cancer, kidney cancer, pancreatic cancer, lung cancer and skin cancer.
